# EUROPÄISCHE PATENTSCHRIFT

(11) **EP 2 906 571 B1**
(45) Veröffentlichungstag und Bekanntmachung des Hinweises auf die Patenterteilung: **24.08.2016**
(21) Anmeldenummer: 13773204.6
(22) Anmeldetag: 27.09.2013
(51) Int. Cl.: C07F 9/6574, B01J 31/02, B01J 31/18, C07C 45/50

(54) **UNSYMMETRISCHES BISPHOSPHIT**
ASYMMETRIC BIPHOSPHITE
BIPHOSPHITE ASYMÉTRIQUE

(30) Priorität: 12.10.2012 DE 102012218627; 12.10.2012 DE 102012218625; 12.10.2012 DE 102012218629; 12.10.2012 DE 102012218630
(43) Veröffentlichungstag der Anmeldung: 19.08.2015
(73) Patentinhaber: Evonik Degussa GmbH, 45128 Essen (DE)
(72) Erfinder: CHRISTIANSEN, Andrea, 18119 Rostock (DE); FRANKE, Robert, 45772 Marl (DE); FRIDAG, Dirk, 45721 Haltern am See (DE); HESS, Dieter, 45770 Marl (DE); DYBALLA, Katrin, Marie, 45657 Recklinghausen (DE); HANNEBAUER, Bernd, 63165 Mühlheim (DE)
(86) Internationale Anmeldenummer: PCT/EP2013/070210
(87) Internationale Veröffentlichungsnummer: WO 2014/056733

(56) Entgegenhaltungen:
- EP-B1- 1 294 731
- DE-A1-102008 002 187
- US-A- 4 769 498

## Beschreibung

Die Erfindung betrifft ein unsymmetrisches Bisphosphit, ein Verfahren zu dessen Herstellung, sowie dessen Umsetzung mit Metallen zu Gemischen enthaltend Komplexverbindungen aus dem unsymmetrischen Bisphosphit und dem Metall sowie deren Verwendung als katalytisch aktive Zusammensetzung in Hydroformylierungsreaktionen, wobei die hydroformylierungsaktive Zusammensetzung neben der Komplexverbindung aus Metall sowie unsymmetrischem Bisphosphit, ungebundenes Bisphosphit und zumindest eine weitere Komponente umfasst.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor P^{III}. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI: 10. 1021 /cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Die Patente US 4 694 109 und US 4 879 416 beschreiben Bisphosphinliganden und ihren Einsatz in der Hydroformylierung von Olefinen bei niedrigen Synthesegasdrücken. Besonders bei der Hydroformylierung von Propen werden mit Liganden dieses Typs hohe Aktivitäten und hohe n/i-Selektivitäten (n/i = das Verhältnis von linearem Aldehyd (=n) zu verzweigtem (=iso) Aldehyd)) erreicht. In WO 95/30680 werden zweizähnige Phosphinliganden und ihr Einsatz in der Katalyse, unter anderem auch in Hydroformylierungsreaktionen, offen gelegt. Ferrocenverbrückte Bisphosphine werden beispielsweise in den Patentschriften US 4 169 861, US 4 201 714 und US 4 193 943 als Liganden für Hydroformylierungen beschrieben.

Der Nachteil von zwei- und mehrzähnigen Phosphinliganden ist ein relativ hoher Aufwand, der zu ihrer Darstellung notwendig ist. Daher ist es oftmals nicht rentabel, solche Systeme in technischen Prozessen einzusetzen. Hinzu kommt eine vergleichsweise geringe Aktivität, die durch hohe Verweilzeiten reaktionstechnisch kompensiert werden muss. Dies wiederum führt zu unerwünschten Nebenreaktionen der Produkte.

Rhodium-Monophosphit-Komplexe in katalytisch aktiven Zusammensetzungen sind geeignet für die Hydroformylierung von verzweigten Olefinen mit innenständigen Doppelbindungen, jedoch ist die n/i-Selektivität für endständig oxierte Verbindungen gering. Aus EP 0 155 508 ist die Verwendung von bisarylensubstituierten Monophosphiten bei der rhodiumkatalysierten Hydroformylierung von sterisch gehinderten Olefinen, z. B. Isobuten, bekannt.

Die in EP 1 294 731 offenbarten Bisphosphite weisen bei der Hydroformylierung von n-Octengemischen Olefinumsätze bis zu 98 % auf. Jedoch ist die ebenfalls gewünschte n/i-Selektivität zum Nonanal mit 36,8 % bis maximal 57,6 % verbesserungswürdig. Dies gilt umso mehr, als dass die Verwendung der katalytisch aktiven Zusammensetzung in technischen Prozessen eine Standzeit verlangt, welche sich in Tagen anstelle von Stunden bemisst.

Literaturbekannt ist die Synthese symmetrisch aufgebauter Bisphosphite, wie sie seit US 4769498 offenbart wurden und deren Verwendung in katalytisch aktiven, übergangsmetallhaltigen Zusammensetzungen zur Hydroformylierung ungesättigter Verbindungen.

In US 4769498, wie auch in US 5723641 werden bevorzugt symmetrisch aufgebaute Bisphosphite hergestellt und als Liganden zur Hydroformylierung verwendet. Die in der Hydroformylierung verwendeten symmetrisch aufgebauten Bisphosphitliganden werden bei tiefen Temperaturen hergestellt. Die Einhaltung dieser tiefen Temperaturen ist zwingend erforderlich, da höhere Temperaturen gemäß dieser US-Schriften zu Umlagerungen und letztlich zu unsymmetrisch aufgebauten Bisphosphiten führen würde, was aber hier nicht erwünscht ist.

In WO95/28228 sowie US5512695 wird die Synthese von unsymmetrischen Bisphosphiten beschrieben. Hierbei wird die Synthese bei Raumtemperatur und/oder bei erhöhter Temperatur durchgeführt.

In WO 95/28228 auf Seite 19 wird die Synthese und die Verwendung des unsymmetrischen Liganden A in der Hydrocyanierung beschrieben, der die unsymmetrische Variante des so genannten (symmetrischen) Biphephosliganden darstellt (siehe unsymmetrisches Isomer des Biphephos).

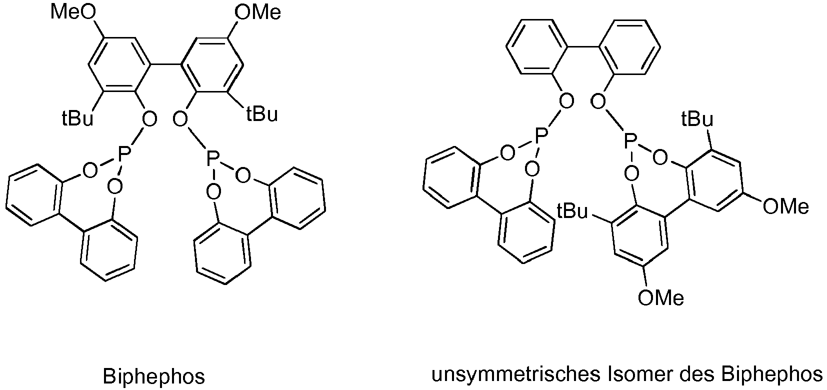

Die Verwendung beider Liganden, also des symmetrischen Biphephosliganden und seines unsymmetrischen Isomers ist ebenfalls in der Hydroformylierung beschrieben. In Rhodium-catalyzed Hydroformylation, ed. by P.W.N.M. van Leeuwen et C. Claver, Kluwer Academic Publishers 2006, AA Dordrecht, NL, Seite 45-46, Tabelle 2 sind die Hydroformylieriungsergebnisse beider Liganden unter vergleichbaren Bedingungen dargestellt. Dabei geht deutlich hervor, dass sich der symmetrische Biphephosligand (in der Literaturstelle Ligand 5a) durch eine deutlich höhere n/i-Selektivität und eine höhere Aktivität auszeichnet als sein unsymmetrisches Isomer (in der Literaturstelle Ligand 7). In der Hydroformylierungsreaktion von Propen weist der symmetrische Ligand eine n/i-Selektivität von 53 und eine Reaktionsrate von 402 auf, wohingegen der unsymmetrische Ligand lediglich eine n/i-Selektivität von 1.2 und eine Reaktionsrate von 280 auf.

Diese unsymmetrisch aufgebauten Bisphosphite weisen bei der Verwendung als Ligand in der übergangsmetallkatalysierten Hydroformylierung somit deutlich geringere Reaktivitäten und geringere n-Regioselektivität auf; siehe in Rhodium-catalyzed Hydroformylation, ed. by P.W.N.M. van Leeuwen et C. Claver, Kluwer Academic Publishers 2006, AA Dordrecht, NL, Seite 45-46.

Wie von van Leeuwen ausgeführt, weisen die symmetrischen Bisphosphite neben höheren n/i-Selektivitäten auch eine größere Reaktivität auf. Neben dem Bestreben einer hohen Reaktivität und n/i-Selektivität in Bezug auf die zu carbonylierenden, ungesättigten Verbindungen ist die Stabilität - konkret die Standzeit - der katalytisch aktiven Zusammensetzung aus jeweils verwendetem Metall, Liganden sowie weiteren Komponenten mit aktivierender Wirkung mit Blick auf die als Liganden eingesetzten Bisphosphite eine ständige Aufgabe der Forschung. Dies gilt insbesondere hinsichtlich olefinhaltiger Gemische, speziell in der Hydroformylierung von Gemischen linearer Olefine.

In US 5364950, wie auch in US 5763677 und in *"*Catalyst Separation, Recovery and Recycling", herausgegeben v. D.J. Cole-Hamilton, R.P. Tooze, 2006, NL, Seiten 25-26, wird die Bildung von sogenannten *"Poisoning Phosphites"* als Neben- bzw. Ligandenabbaureaktion beschrieben. Diese *"Poisoning Phosphites"* bilden sich bei der Verwendung von arylphosphit-modifizierten Rhodium-Komplexen während der Hydroformylierungsreaktion. Hierbei kommt es im Zuge des Ligandenabbaus zu einem Austausch einer Arylgruppe durch eine Alkylgruppe des Hydroformylierungsproduktes.

Neben der Bildung der unerwünschten *"Poisoning Phosphites"* kann der Phosphitligand auch im Zuge einer Hydrolysereaktion durch die bei der Aldehydkondensation gebildeten Wasserspuren abgebaut werden. Eine Konsequenz aus diesen Abbaureaktionen der Liganden ist, dass die Konzentration an hydroformylierungsaktiven Rhodiumkomplexspezies im Laufe der Zeit abnimmt und mit einem Verlust an Reaktivität einher geht. Es ist allgemein bekannt, dass bei einer kontinuierlichen Fahrweise der Hydroformylierung Ligand/en und - gegebenenfalls weitere Komponenten - während des Reaktionsverlaufs nachdosiert, d. h. nach Reaktionsbeginn zusätzlich zugegeben werden müssen (siehe DE 10 2008 002 187 A1).

Die technische Aufgabe der Erfindung ist die Bereitstellung eines neuen Liganden, welcher in der Hydroformylierung von ungesättigten Verbindungen nicht die aus dem Stand der Technik zuvor aufgezeigten Nachteile, sondern die folgenden Eigenschaften aufweist:
1) eine hohe Aktivität,
2) eine hohe n-Regioselektivität in Bezug auf die Hydroformylierung,
3) eine hohe Standzeit und Langzeitstabilität.

Eine hohe Standzeit bedeutet, dass die hydroformylierungsaktive Zusammensetzung, welche den Liganden neben weiteren Komponenten umfasst, eine geringe Tendenz zum Abbau dieses Liganden und/oder den Zerfall dieses Liganden in hydroformylierungsinhibierende Komponenten, wie z.B. die sogenannten *"Poisoning Phosphites"* aufweist.

Die Aufgabe wird gelöst durch eine Verbindung gemäß Formel (1):

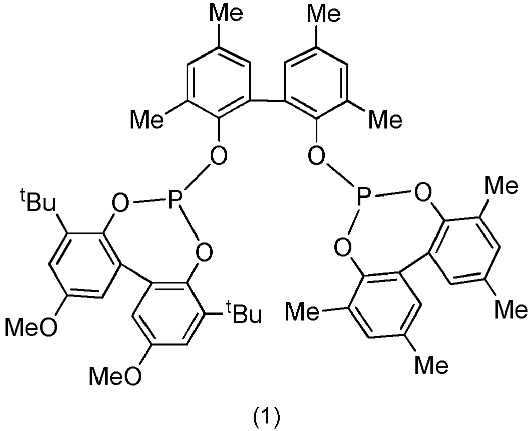

Die Erfindung umfasst folgende Gegenstände:
a) ein unsymmetrisch aufgebautes Bisphosphit;
b) Verfahren zu dessen Herstellung
c) Gemische enthaltend zumindest eine Komplexverbindung der Formel (2), wobei M ein Metall der 4. bis 10. Gruppe des Periodensystems der Elemente (Fe, Ru, Os, Co, Rh, Ir, Ni, Pd, Pt) darstellt und zusätzliche Bindungen eingehen kann sowie das unter a) genannte Bisphosphit, welches nicht an das Metall M gebunden ist. 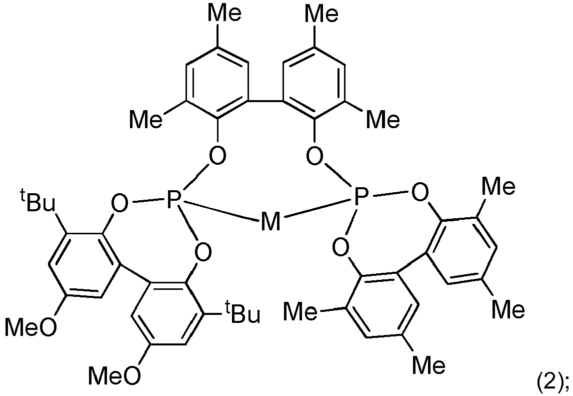
d) Zusammensetzungen, enthaltend das unter a) genannte Bisphosphit, Metalle der 4. bis 10. Gruppe des Periodensystems der Elemente (Fe, Ru, Os, Co, Rh, Ir, Ni, Pd, Pt) sowie freies, d.h. ungebundenes Bisphosphit und zumindest eine weitere Komponente, ausgewählt aus der Gruppe, welche Basen, organische Amine, Epoxide, Ionenaustauscher, Puffersysteme umfasst. ;
e) Verfahren zur Hydroformylierung ungesättigter Verbindungen und deren Gemischen unter Verwendung von Zusammensetzungen nach d), eines Gasgemisches bestehend aus Kohlenmonoxid und Wasserstoff, ungesättigter Verbindungen und deren Gemischen unter den für eine Hydroformylierung erforderlichen Reaktionsbedingungen;
f) Mehrphasiges Reaktionsgemisch bestehend aus:
   f1) mindestens einer Zusammensetzung nach d);
   f2) einem Gasgemisch bestehend aus Kohlenmonoxid und Wasserstoff;
   f3) mindestens einer ungesättigten Verbindung als Substrat;
   f4) mindestens einem Hydroformylierungsprodukt aus den Substraten.

Das erfindungsgemäße Verfahren zur Herstellung des unsymmetrischen Bisphosphits (1) umfasst die Schritte::
i) oxidative Kopplung von 2,4-Dimethylphenol zu 3, 3', 5, 5'-Tetramethyl-2,2'-dihydroxybiphenyl;
ii) oxidative Kopplung von 3-tert.-Butyl-4-Hydroxyanisol zu 5, 5'-Dimethoxy-3, 3'-di-tert.-Butyl-2, 2'-dihydroxybiphenyl;
iii) Umsetzung von 3, 3', 5, 5'-Tetramethyl-2,2'-dihydroxybiphenyl aus i) mit PCl₃ zum Phosphorochloriditderivat unter Inertgasatmosphäre;
iv) Umsetzung von zumindest 2 Äquivalenten des Phophorochloriditderivats aus iii) mit 1 Äquivalent des 5, 5'-Dimethoxy-3, 3'-di-tert.-Butyl-2, 2'-dihydroxybiphenyl aus ii) unter Inertgasatmosphäre.

In einer Variante des Verfahrens wird im Verfahrensschritt iv) ein Lösungsmittelgemisch verwendet.

In einer Variante des Verfahrens erfolgt die Umsetzung in Verfahrensschritt iv) unter Verwendung eines aprotischen Lösungsmittelgemisch ausgewählt aus organischen Stickstoffverbindungen, organischen Estern, Aromaten.

In bevorzugten Varianten des Verfahrens ist die organische Stickstoffverbindung eine Verbindung ausgewählt unter Nitrilen, Aminen, Amiden.

In besonders bevorzugten Varianten des Verfahrens wird ein Lösungsmittel im Verfahrensschritt iv) verwendet, welches ausgewählt ist aus Acetonitril, Triethylamin, Dimethylaminobutan, Di-iso-propylethylamin, N-Methylpyrrolidon, Pyridin, Ethylacetat, Toluol.

In besonders bevorzugten Varianten des Verfahrens erfolgt der Verfahrensschritt iv) in einem aprotisch-polaren Lösungsmittel, oder einem Gemisch, welches mindestens ein aprotisch-polares Lösungsmittel umfasst.

Unter dem Begriff aprotisches Lösungsmittel werden im Rahmen dieser Anmeldung nichtwäßrige Lösemittel, die kein ionisierbares Proton im Molekül enthalten, verstanden, welche weiter in aprotisch-unpolare und aprotisch-polare Lösungsmittel unterteilt sind (siehe Thieme Römpp online).
Unter der Bezeichnung aprotisch-unpolare oder apolar aprotische Lösungsmittel werden aliphatische und aromatische sowie halogenierte Kohlenwasserstoffe (Alkane, Alkene, Alkine, Benzol, Aromaten mit aliphatischen oder aromatischen Seitenketten), perhalogenierte Kohlenwasserstoffe wie Tetrachlorkohlenstoff und Hexafluorbenzol, Tetramethylsilan und Schwefelkohlenstoff zusammengefasst.

Aprotisch-unpolare Lösemittel sind durch niedrige relative Permittivitäten (εr <15), niedrige Dipolmomente (µ <2,5 D) und niedrige ETN-Werte (0,0-0,3; ETN = normalisierte Werte der empirischen Parameter der Lösemittelpolarität) charakterisiert. Aprotisch-unpolare Lösemittel sind lipophil und hydrophob. Zwischen ihren Molekülen herrschen Van-der-Waals-Wechselwirkungen.

Die unter dem Begriff aprotisch-polare oder dipolar aprotische Lösungsmittel zusammengefassten Lösungsmittel besitzen stark polarisierende funktionelle Gruppen und zeigen daher ein gewisses permanentes Dipolmoment, das zu den nun untergeordneten Van-der-Waals-Wechselwirkungen hinzukommt. Ihr Lösevermögen für polare Stoffe ist somit in der Regel besser als das der aprotisch-unpolaren Lösungsmittel. Beispiele aprotischpolarer Lösungsmittel sind Ketone wie Aceton, Ether, Ester, *N,N*-disubstituierte Amide wie Dimethylformamid, tertiäre Amine, Pyridin, Furan, Thiophen, 1,1,1-Trichlorethan, Nitroalkane wie Nitromethan, Nitrile wie Acetonitril, Sulfoxide wie Dimethylsulfoxid, Sulfone, Hexamethylphosphorsäuretriamid, flüssiges Schwefeldioxid, Selenoxychlorid. Diese besitzen große Permittivitäten (εr >15), Dipolmomente (µ >2,5 D) und ETN-Werte im Bereich von 0,3-0,5.

Eine Variante des erfindungsgemäßen Verfahrens umfasst den zusätzlichen Verfahrensschritt v), indem die Verbindung (1) als Feststoff abgetrennt und in einem aprotischen Lösungsmittelgemisch suspendiert wird. In einer weiteren Variante des Verfahrensschritts v) wird die als Feststoff abgetrennte Verbindung (1) in einem aprotischen Lösungsmittelgemisch umkristallisiert.

In besonders bevorzugten Varianten des erfindungsgemäßen Verfahrens erfolgt Ver fahrensschritt v) Suspendieren in Acetonitril bei 75 °C oder in Toluol bei 35 °C.

In besonders bevorzugten Varianten des erfindungsgemäßen Verfahrens erfolgt Ver fahrensschritt v) Umkristallisation in einem aprotischen Lösungsmittelgemisch bestehend aus Toluol/Heptan oder Xylol/Heptan.

Neben der Verbindung gemäß der Formel (1) wird auch eine Verbindung gemäß der Formel (2) beansprucht. Diese umfasst die Verbindung gemäß der Formel (1). Verbindung gemäß der Formel (2): 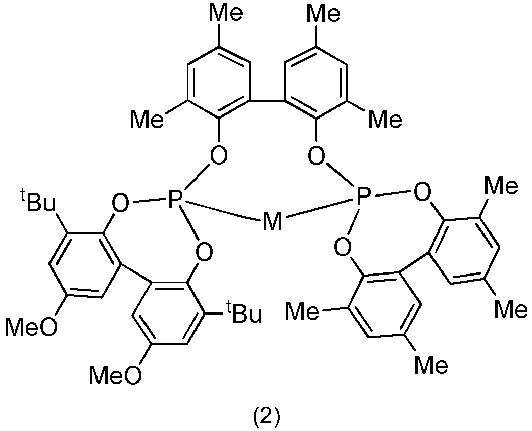 wobei M ausgewählt ist aus Fe, Ru, Os, Co, Rh, Ir, Ni, Pd, Pt und M zusätzliche Bindungen eingehen kann.

Hierbei sind Co, Rh, Ru, Ir, Fe bevorzugt; und Rh besonders bevorzugt.

Die Verbindung gemäß der Formel (2) wird in-situ während der Hydroformylierung gebildet, wie es in den Beispielen offenbart wird.

In einer besonderen Ausführungsform der Erfindung liegt die Verbindung gemäß der Formel (3) vor: 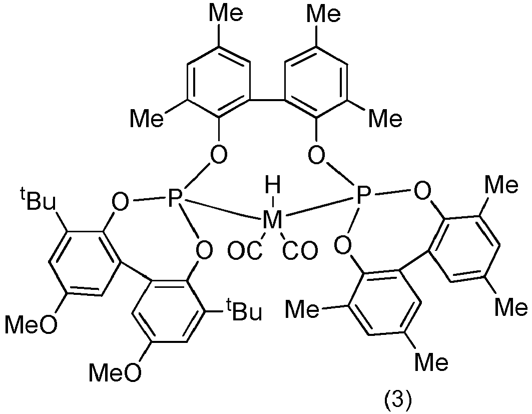 wobei M ausgewählt ist aus: Fe, Ru, Os, Co, Rh, Ir, Ni, Pd, Pt.

Hierbei sind Co, Rh, Ru, Ir, Fe bevorzugt; und Rh besonders bevorzugt.

Neben den reinen Verbindungen werden auch Gemische beansprucht, welche diese umfassen.

Gemische umfassen eine Verbindung gemäß der Formel (2) und/oder (3), wobei das Gemisch zusätzliche eine Verbindung gemäß der Formel (1) umfasst, welche nicht an M koordiniert ist.

Neben den Gemischen werden auch Zusammensetzungen beansprucht.

Die Zusammensetzungen umfassen ein zuvor beschriebenes Gemisch, welche zusätzlich zu dem Gemisch eine weitere Komponente aufweisen ausgewählt aus Basen, organische Amine, Pufferlösungen, Ionenaustauscher, Epoxide.
In US 4567306, US 5364950, US 5741942 und US 5763677 werden Beispiele für diese weiteren Komponenten offenbart.

In einer bevorzugten Ausführungsform werden als weitere Komponenten sterisch gehinderte sekundäre Amine Verbindungen mit der allgemeinen Formel I eingesetzt, 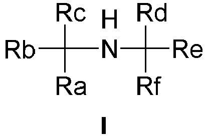 wobei Ra, Rb, Rc, Rd, Re und Rf gleiche oder unterschiedliche Kohlenwasserstoffreste die auch untereinander verbunden sein können, sind.

In einer bevorzugten Ausführungsform weist das organische Amin eine Struktur gemäß Formel **Ia** auf: 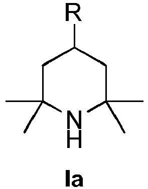 mit R gleich H, wie das 2,2,6,6-Tetramethylpiperidin selbst, einem organischen Rest R, einer Hydroxylgruppe oder einem Halogen.

Der organische Rest R kann auch ein über ein Heteroatom, beispielsweise ein Sauerstoffatom, an die 2,2,6,6-Tetramethylpiperidin-Struktureinheit gebundener, organischer Rest sein. Insbesondere kann der organische Rest polymere Strukturen aufweisen oder ein 1 bis 50 Kohlenstoffatome und gegebenenfalls Heteroatome aufweisender organischer Rest sein. Besonders bevorzugt weist der organische Rest Carbonylgruppen, wie Keto-, Ester- oder Säureamid-Gruppen auf. Der organische, gegebenenfalls Heteroatome aufweisende Rest kann insbesondere ein substituierter oder unsubstituierter, aliphatischer, alicyclischer, aliphatisch-alicyclischer, heterocyclischer, aliphatischheterocyclischer, aromatischer, aromatisch-aromatischer oder aliphatisch-aromatischer Kohlenwasserstoffrest mit 1 bis 50 Kohlenstoffatomen sein, wobei die substituierten Kohlenwasserstoffreste Substituenten, ausgewählt aus primären, sekundären oder tertiären Alkylgruppen, alicyclischen Gruppen, aromatischen Gruppen, -N(R¹)₂, -NHR¹,-NH₂, Fluor, Chlor, Brom, Jod, -CN, -C(O)-R¹, -C(O)H oder -C(O)O-R¹, -CF₃, -O-R¹, - C(O)N-R¹, -OC(O)-R¹ und/oder -Si(R¹)₃, mit R¹ gleich einem monovalenten, bevorzugt 1 bis 20 Kohlenstoffatome aufweisenden Kohlenwasserstoffrest, aufweisen können. Sind mehrere Kohlenwasserstoffreste R¹ vorhanden, so können diese gleich oder unterschiedlich sein. Die Substituenten sind vorzugsweise beschränkt auf solche, die keinen Einfluss auf die Reaktion selbst haben. Besonders bevorzugte Substituenten können ausgewählt sein aus den Halogenen, wie z. B. Chlor, Brom oder Jod, den Alkylresten, wie z. B. Methyl, Ethyl, Propyl, iso-Propyl, Butyl, sec-Butyl, t-Butyl, neo-Pentyl, sec-Amyl, t-Amyl, iso-Octyl, t-Octyl, 2-Ethylhexyl, iso-Nonyl, iso-Decyl oder Octadecyl, den Arylresten, wie z. B. Phenyl, Naphthyl oder Anthracyl, den Alkylarylresten, wie z. B. Tolyl, Xylyl, Dimethylphenyl, Diethylphenyl, Trimethylphenyl, Triethylphenyl oder p-Alkylphenyl, den Aralkylresten, wie z. B. Benzyl oder Phenylethyl, den alicyclischen Resten, wie z. B. Cyclopentyl, Cyclohexyl, Cyclooctyl, Cyclohexylethyl oder 1-Methylcyclohexyl, den Alkoxyresten, wie z. B. Methoxy, Ethoxy, Propoxy, Butoxy oder Pentoxy, den Aryloxyresten, wie z. B. Phenoxy oder Naphthoxy, -OC(O)R¹ oder -C(O)R¹, wie z. B. Acetyl, Propionyl, Trimethylacetoxy, Triethylacetoxy oder Triphenylacetoxy, und den drei Kohlenwasserstoffreste aufweisenden Silylresten -Si(R¹)₃, wie z. B. Trimethylsilyl, Triethylsilyl oder Triphenylsilyl. Besonders bevorzugt sind Verbindungen der Formel IIa, die als Reste R solche aufweisen, die einen 2,2,6,6,-Tetramethylpiperidin-Rest und gegebenenfalls eine weitere -N(R¹)₂, -NHR¹ und/oder-NH₂ Gruppe enthalten.

Als sekundäre Amine, die eine Struktureinheit gemäß Formel I aufweisen, können ganz besonders bevorzugt die nachfolgend aufgeführten Verbindungen mit den Strukturformeln **Ib** bis **Ig** oder deren Derivate eingesetzt werden. 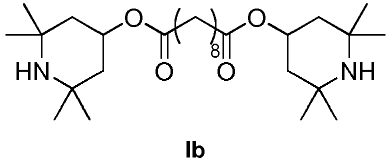 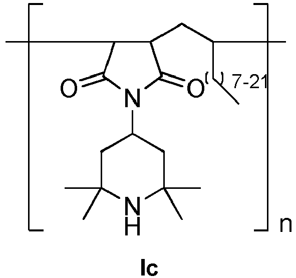 mit n = 1 bis 20, vorzugsweise 1 bis 10 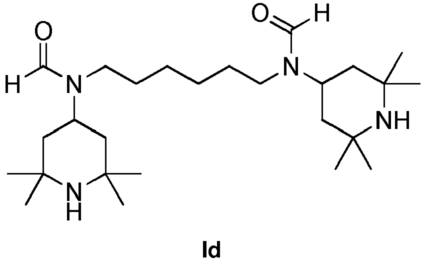 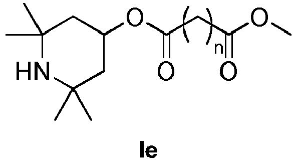 mit n = 1 bis 12, vorzugsweise 8 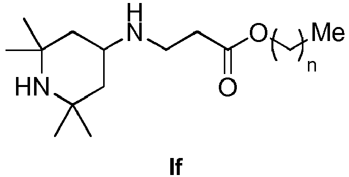 mit n = 1 bis 17, vorzugsweise 13

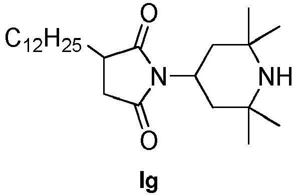

Es können auch Gemische, enthaltend zwei oder mehrere sterisch gehinderte Amine, eingesetzt werden. Die Zusammensetzung umfasst ein zuvor beschriebenes Gemisch, welche zusätzlich zu dem Gemisch zumindest ein Amin mit einer 2,2,6,6-Tetramethylpiperidineinheit aufweist. Insbesondere wird im erfindungsgemäßen Verfahren das Amin mit der Formel **Ib,** Sebacinsäuredi-4-(2,2,6,6-tetramethylpiperidinyl)ester, bevorzugt eingesetzt.
Ein besonders bevorzugtes Metall in der erfindungsgemäßen Zusammensetzung ist Rhodium.

Des Weiteren wird ein Verfahren zur Hydroformylierung von ungesättigten Verbindungen und deren Gemischen beansprucht, welches diese Zusammensetzungen verwendet.

Verfahren zur Hydroformylierung einer ungesättigten Verbindung oder eines Gemisches von ungesättigten Verbindungen umfassend die Verfahrensschritte:
a) Vorlegen einer Verbindung nach den Formeln (1), (2) und/oder (3) oder Zusammensetzung enthaltend die Verbindungen der Formeln (1), (2) und (3) zusammen mit einer weiteren Komponente ausgewählt aus Basen, organischen Aminen, Pufferlösungen, Ionenaustauschern, Epoxiden,
b) Einleiten eines Gasgemisches umfassend Kohlenmonoxid und Wasserstoff,
c) Zugabe mindestens einer ungesättigten Verbindung oder eines Gemisches von ungesättigten Verbindungen.

Die ungesättigten Verbindungen, welche in dem erfindungsgemäßen Verfahren hydroformyliert werden, umfassen Kohlenwasserstoffgemische, die in petrochemischen Verarbeitungsanlagen anfallen. Hierzu gehören beispielsweise sogenannte C₄-Schnittte. Typische Zusammensetzungen von C₄-Schnitten, aus denen der größte Teil der mehrfach ungesättigten Kohlenwasserstoffe entfernt worden ist und die im erfindungsgemäßen Verfahren eingesetzt werden können, sind in der folgenden Tabelle 1 aufgelistet (siehe DE 10 2008 002188).

**Tabelle 1:**

| | Dampfspaltanlage | | Dampfspaltanlage | | Katalytische Spaltanlage | |
|---|---|---|---|---|---|---|
| Komponente | HCC₄ | HCC₄ / SHP | Raff. I | Raff. I / SHP | CC₄ | CC₄ / SHP |
| Isobutan [Massen-%] | 1 - 4.5 | 1 - 4.5 | 1.5 - 8 | 1.5 - 8 | 37 | 37 |
| n-Butan [Massen-%] | 5-8 | 5-8 | 6-15 | 6-15 | 13 | 13 |
| E-2-Buten [Massen-%] | 18-21 | 18-21 | 7-10 | 7-10 | 12 | 12 |
| 1-Buten [Massen-%] | 35-45 | 35-45 | 15-35 | 15-35 | 12 | 12 |
| Isobuten [Massen-%] | 22-28 | 22-28 | 33-50 | 33-50 | 15 | 15 |
| Z-2-Buten [Massen-%] | 5-9 | 5-9 | 4-8 | 4-8 | 11 | 11 |
| 1,3-Butadien [Massen-ppm] | 500 - 8000 | 0-50 | 50 - 8000 | 0-50 | < 10000 | 0-50 |

### Erläuterung:

- HCC₄: typisch für eine C₄ Mischung, die aus dem C₄-Schnitt einer Dampfspaltanlage (High Severity) nach der Hydrierung des 1,3-Butadiens ohne zusätzliche Moderation des Katalysators erhalten wird.
- HCC₄ / SHP: Zusammensetzung HCC₄, bei dem Reste an 1,3-Butadien in einem Selektivhydrierungsprozess/SHP weiter reduziert wurden.
- Raff. I (Raffinat I): typisch für eine C₄ Mischung, die aus dem C₄-Schnitt einer Dampfspaltanlage (High Severity) nach der Abtrennung des 1,3-Butadiens, beispielsweise durch eine NMP-Extraktivrektifikation, erhalten wird.
- Raff. I / SHP: Zusammensetzung Raff. I, bei dem Reste an 1,3-Butadien in einem Selektivhydrierungsprozess/SHP weiter reduziert wurden.
- CC₄: typische Zusammensetzung eines C₄-Schnitts, das aus einer katalytischen Spaltanlage erhalten wird.
- CC₄ / SHP: Zusammensetzung eines C₄-Schnitts, bei dem Reste an 1,3-Butadien in einem Selektivhydrierungsprozess/SHP weiter reduziert wurden.

In einer Variante des Verfahrens ist die ungesättigte Verbindung oder deren Gemisch unter c) ausgewählt aus:
- Kohlenwasserstoffgemischen aus Dampfspaltanlagen;
- Kohlenwasserstoffgemischen aus katalytisch betriebenen Spaltanlagen, wie z.B. FCC-Spaltanlagen;
- Kohlenwasserstoffgemischen aus Oligomerisierungsprozessen in homogener Phase sowie heterogenen Phasen, wie z.B. dem OCTOL-, DIMERSOL.-, Fischer-Tropsch-, Polygas-, CatPoly-, InAlk-, Polynaphtha-, Selectopol-, MOGD-, COD-, EMOGAS-, NExOCTANE- oder SHOP-Prozess;
- Kohlenwasserstoffgemischen umfassend mehrfach ungesättigte Verbindungen;
- ungesättigten Carbonsäurederivaten.

In einer Variante des Verfahrens weist das Gemisch ungesättigte Verbindungen mit 2 bis 30 Kohlenstoffatomen auf.

In einer Variante des Verfahrens weist das Gemisch ungesättigte Verbindungen mit 2 bis 8 Kohlenstoffatomen auf.

In einer weiteren Variante des Verfahrens weist das Gemisch mehrfach ungesättigte Kohlenwasserstoffe auf. In einer besonderen Ausführungsform umfasst das Gemisch Butadiene.

Die ungesättigten Verbindungen, welche in dem erfindungsgemäßen Verfahren hydroformyliert werden, umfassen weiterhin ungesättigte Carbonsäurederivate. In einer besonderen Ausführungsform sind diese ungesättigten Carbonsäurederivate ausgewählt unter Fettsäureestern.

Die Durchführung des erfindungsgemäßen Verfahrens erfolgt in unterschiedlichen Ausführungsformen, welche in den Beispielen im Detail offenbart werden.

Das erfindungsgemäße mehrphasige Reaktionsgemisch umfasst neben einem aus Kohlenmonoxid und Wasserstoff bestehenden Gasgemisch mindestens eine ungesättigte Verbindung, wie sie zuvor offenbart wurde und umfasst neben Kohlenwasserstoffgemischen, welche aus Dampfspalt-, katalytisch betriebenen Spaltanlagen oder Oligomerisierungsprozessen stammen oder andere Quellen von einfach ungesättigten und/oder mehrfach ungesättigten Kohlenstoffverbindungen oder ungesättigte Carbonsäurederivate beinhalten, mindestens ein Hydroformylierungsprodukt dieser ungesättigten Verbindungen, wie sie in den nachfolgenden Beispielen aufgeführt sind und die jeweils verwendete Zusammensetzung, wie sie zuvor offenbart wurde.

### Figurenbeschreibung: Berechnung der Komplexverbindung (3)

Die erfindungsgemäßen Komplexverbindungen der Formeln (2) und (3) werden in-situ während der Hydroformylierungsreaktion gebildet.

In einer besonderen Ausführungsform der Erfindung liegen die Komplexverbindungen (2) und (3) neben dem ungebundenen Bisphosphit (1) vor.

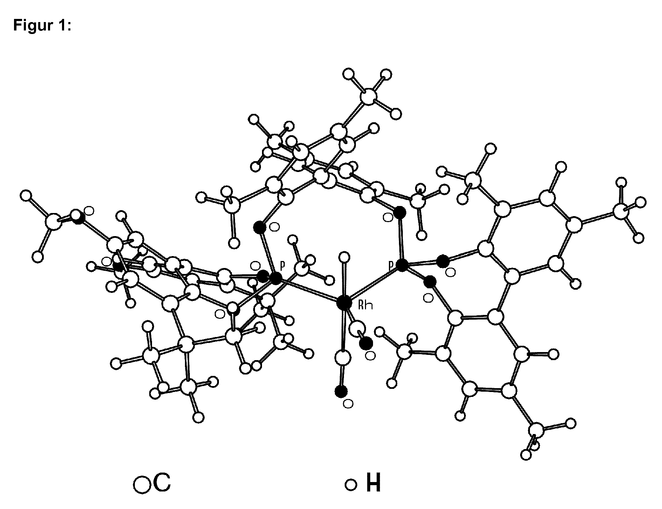

Die Charakterisierung des Hydridocarbonylkomplexes des Liganden (1) mit Rhodium als Metall, der erfindungsgemäßen Verbindung (3), erfolgte mittels theoretischer Berechnungen. Das Ergebnis ist in der Figur 1 im Anhang dargestellt.

Die Strukturberechnung wurde mit dem BP86-Funktional und dem def-SV(P)-Basissatz durchgeführt.
Die Strukturberechnungen für die Modellstrukturen erfolgten mit dem Turbomole-Programmpaket (R. Ahlrichs, M. Bär, M. Häser, H. Horn, C. Kölmel, Chem. Phys. Lett., 1989, 162, 16; TURBOMOLE V6.3 2011, a development of University of Karlsruhe and Forschungszentrum Karlsruhe GmbH, 1989-2007, TURBOMOLE GmbH, since 2007. http://www.turbomole.com) auf Basis der Dichtefunktionaltheorie (DFT). Verwendet wurde das BP86-Funktional (S. H. Vosko, L. Wilk, M. Nusair, Can. J. Phys. , 1980, 58, 1200; A. D. Becke, Phys. Rev. A, 1988, 38, 3098; J. Perdew, Phys. Rev. B, 1986, 33, 8822) und der def-SV(P)-Basissatz (A. Schäfer, H. Horn and R. Ahlrichs, J. Chem. Phys., 1992, 97, 2571).

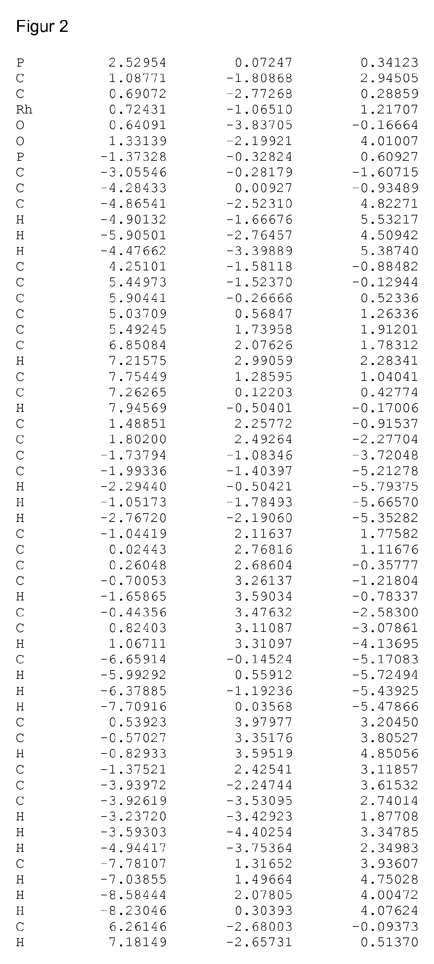

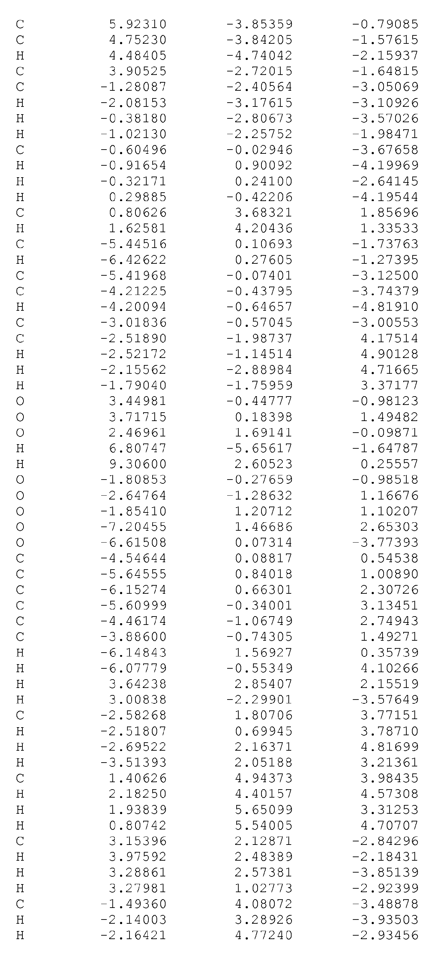

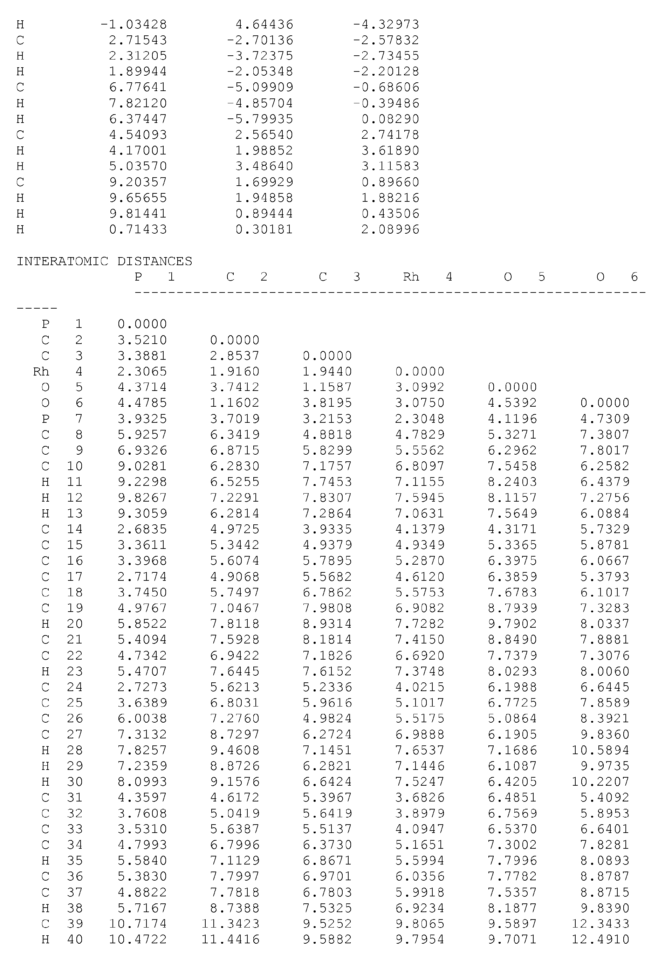

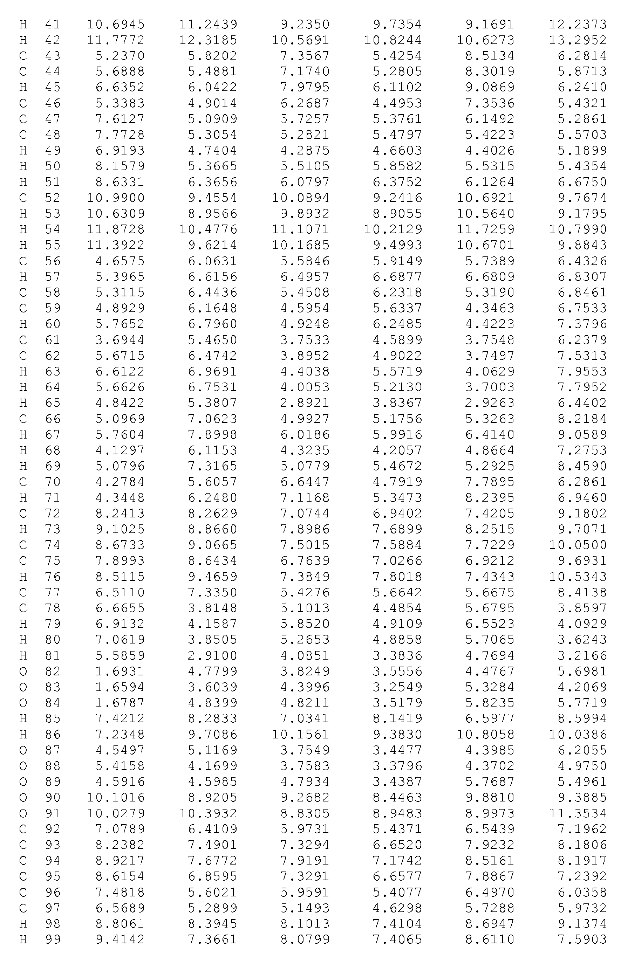

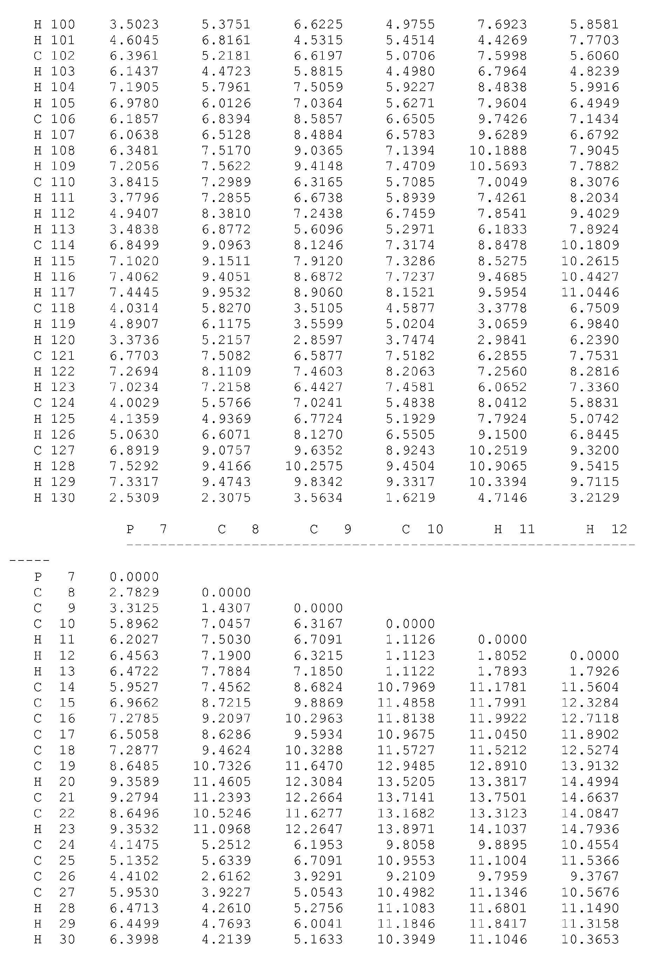

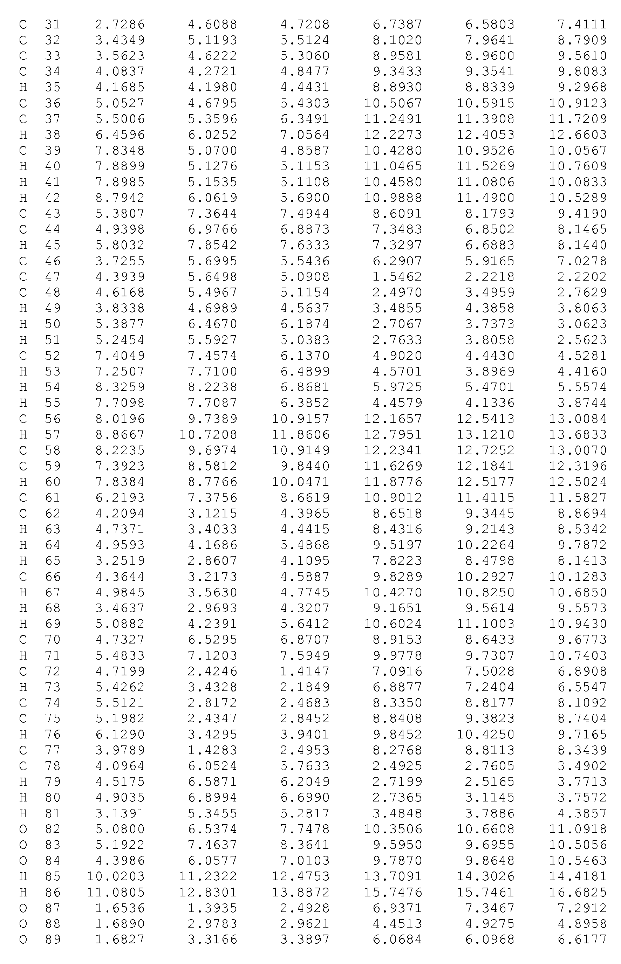

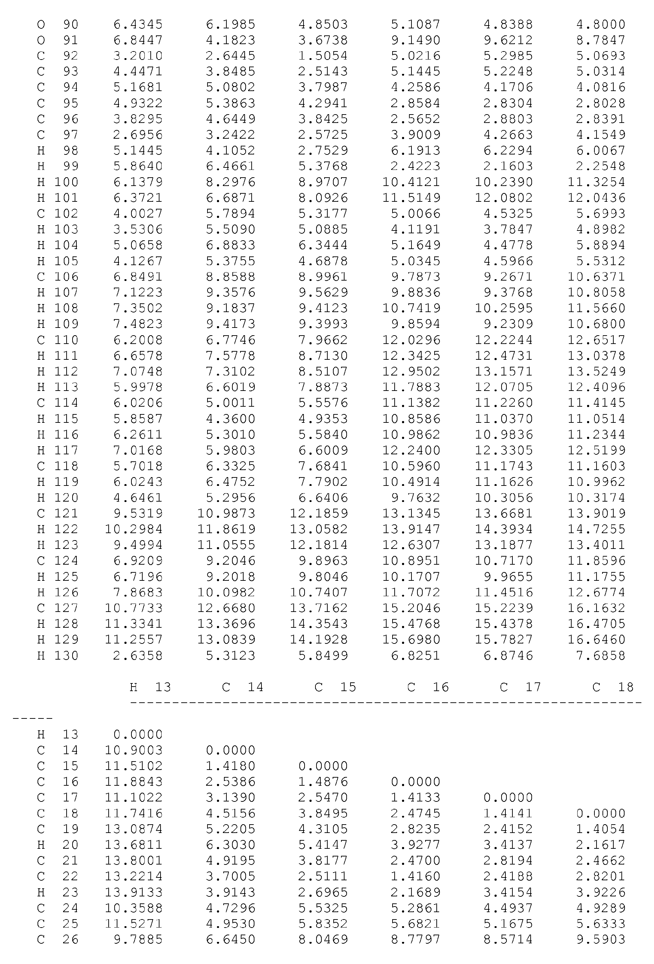

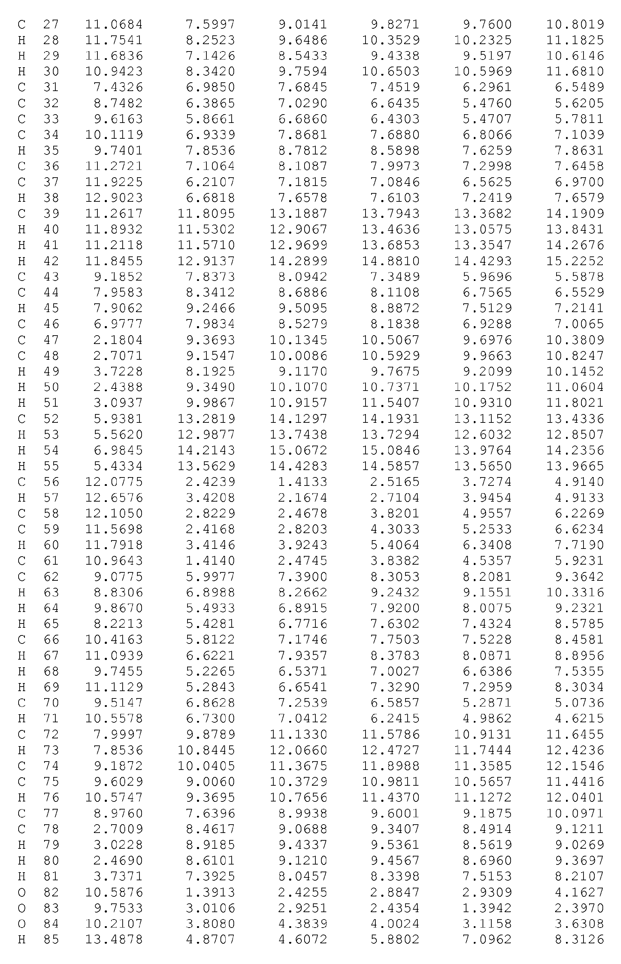

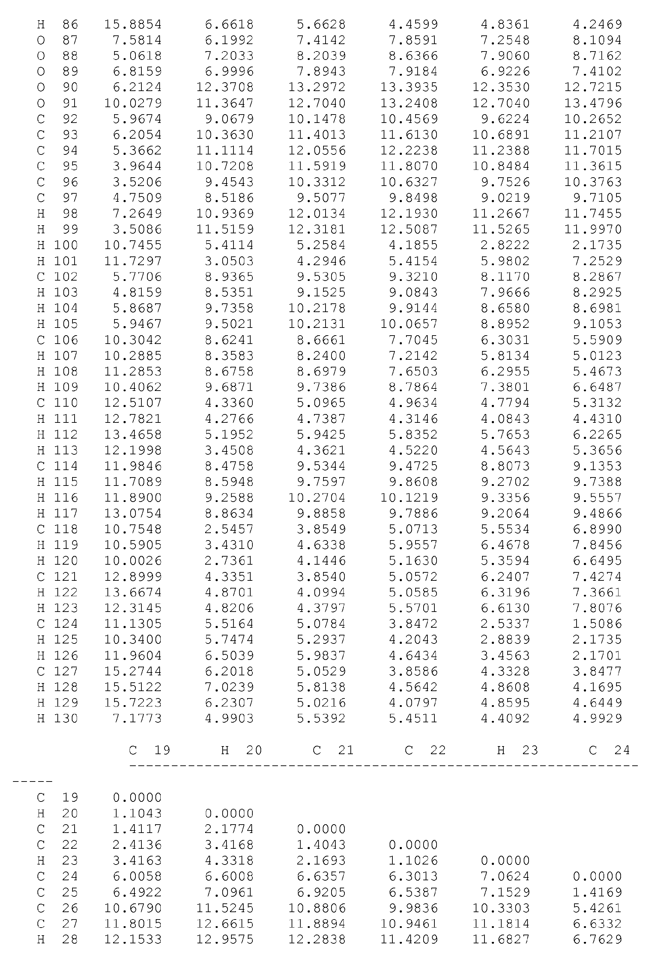

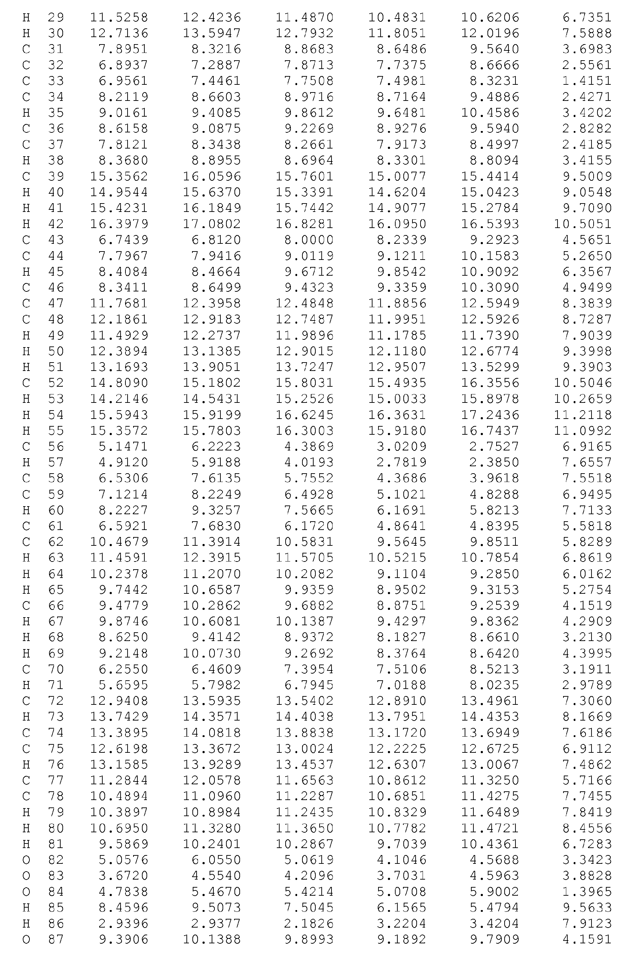

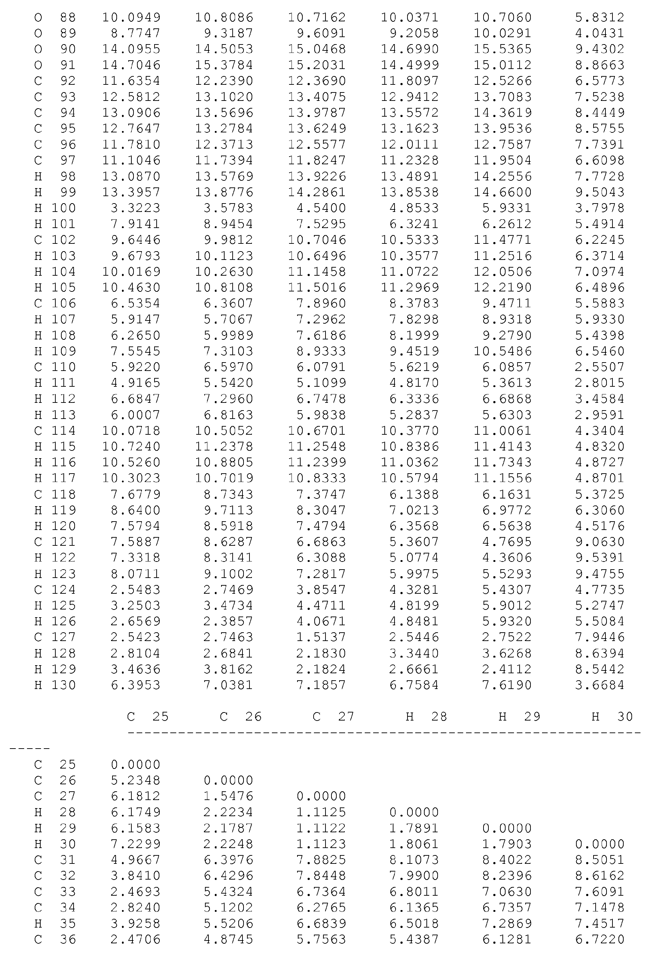

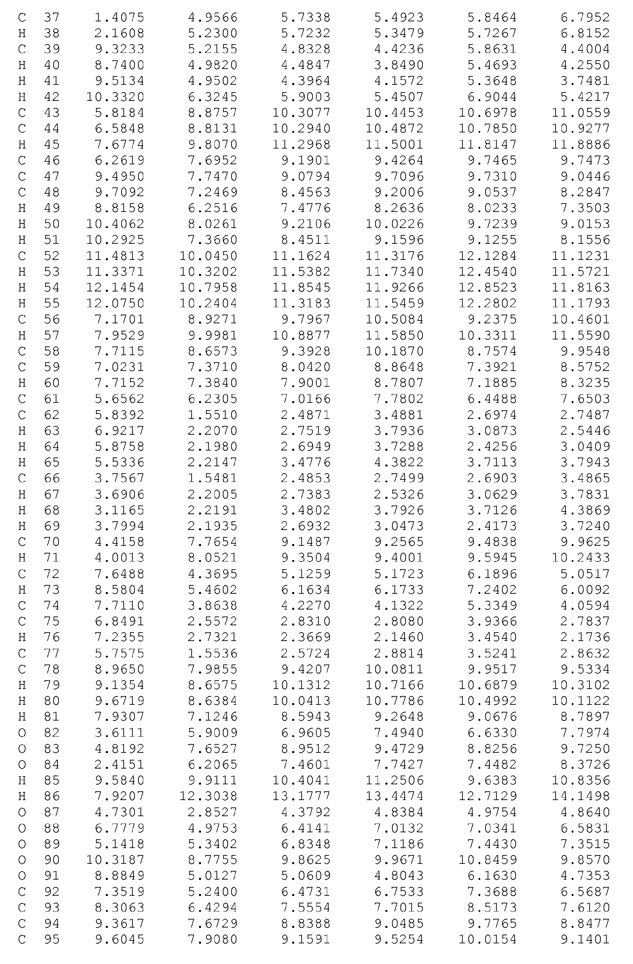

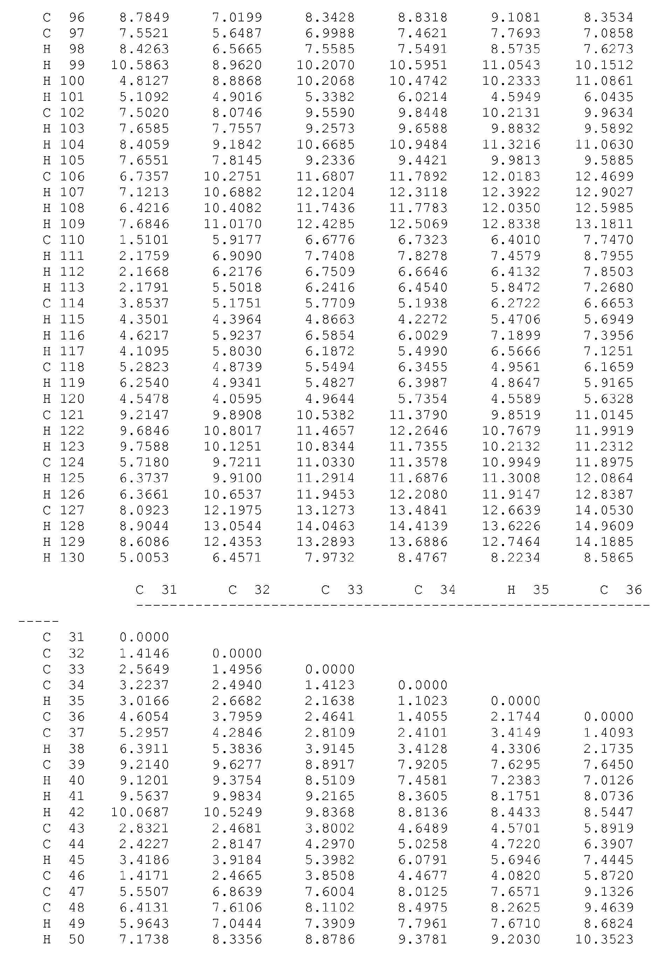

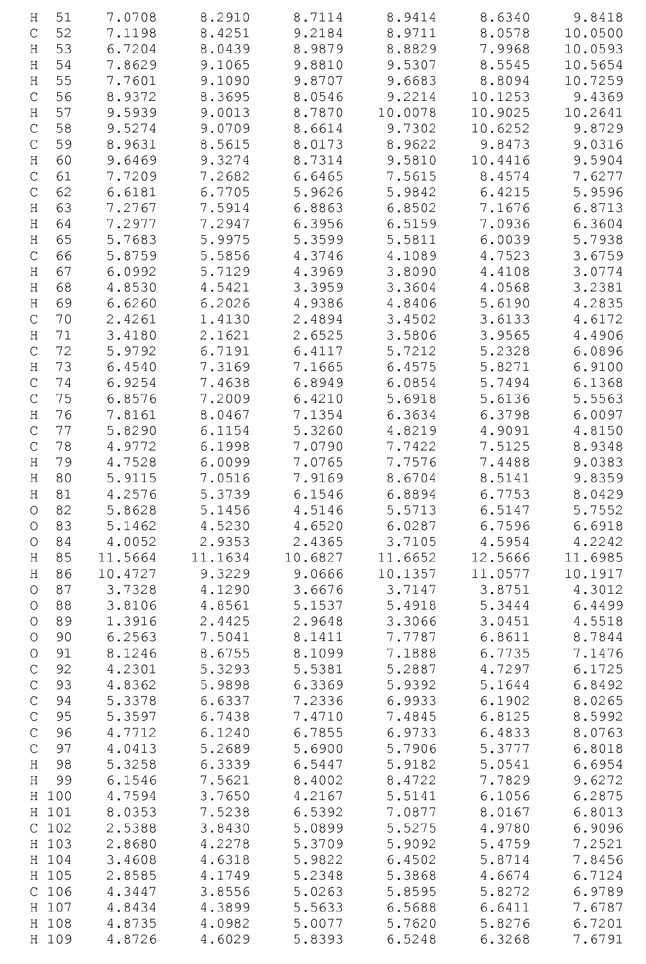

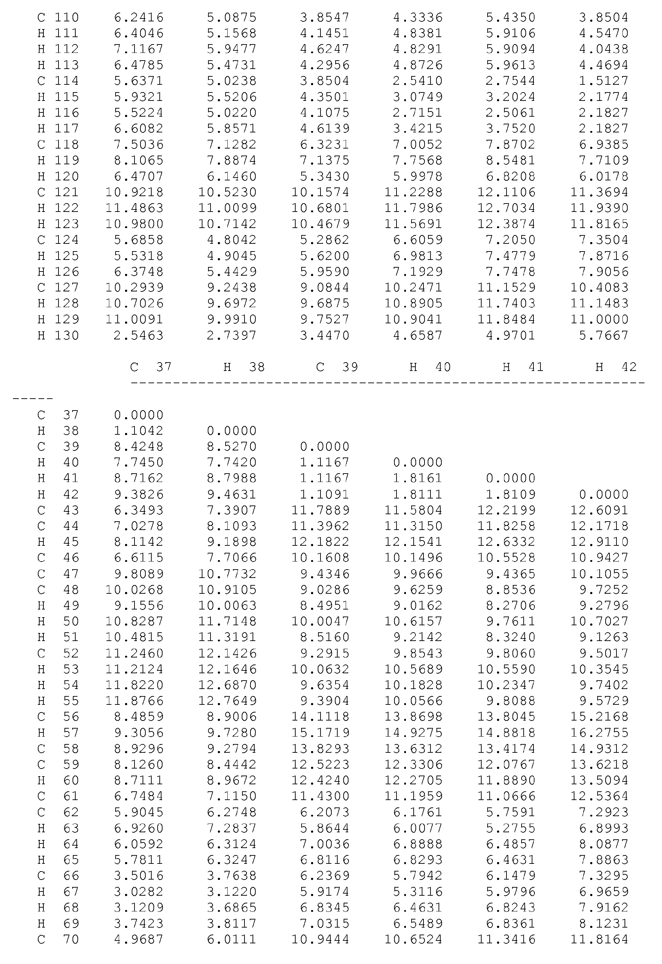

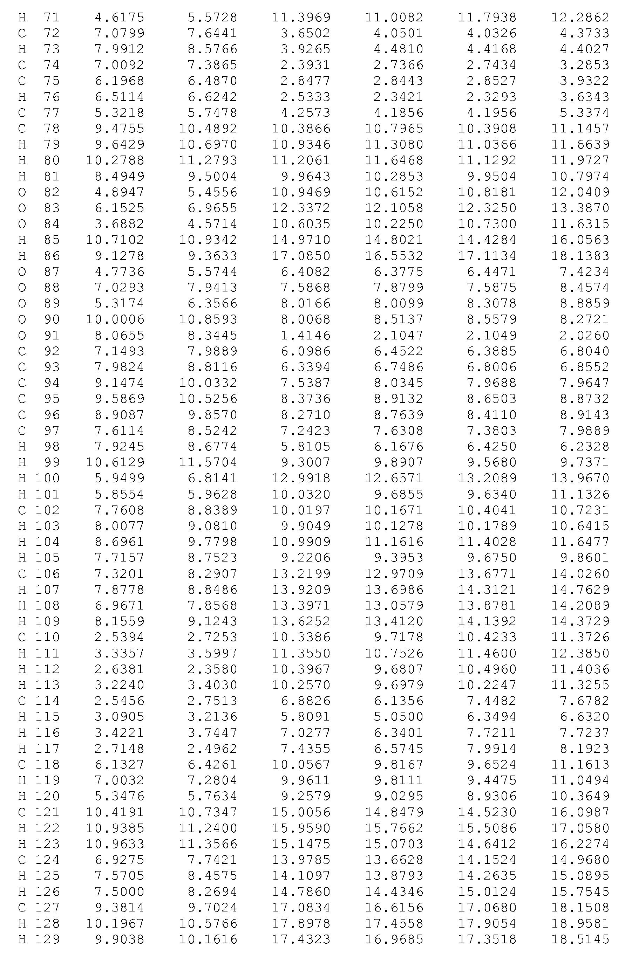

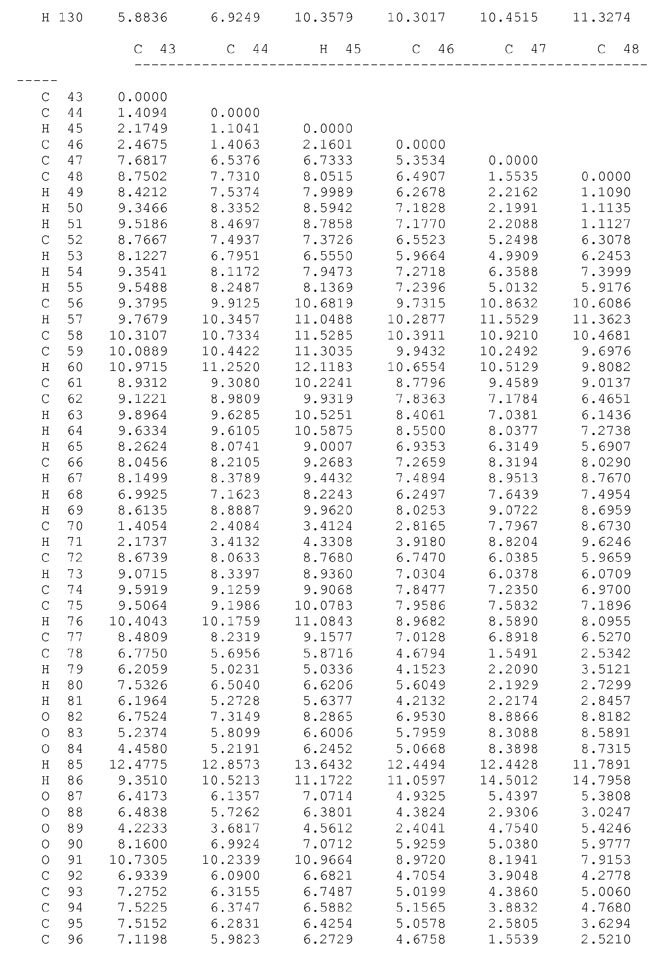

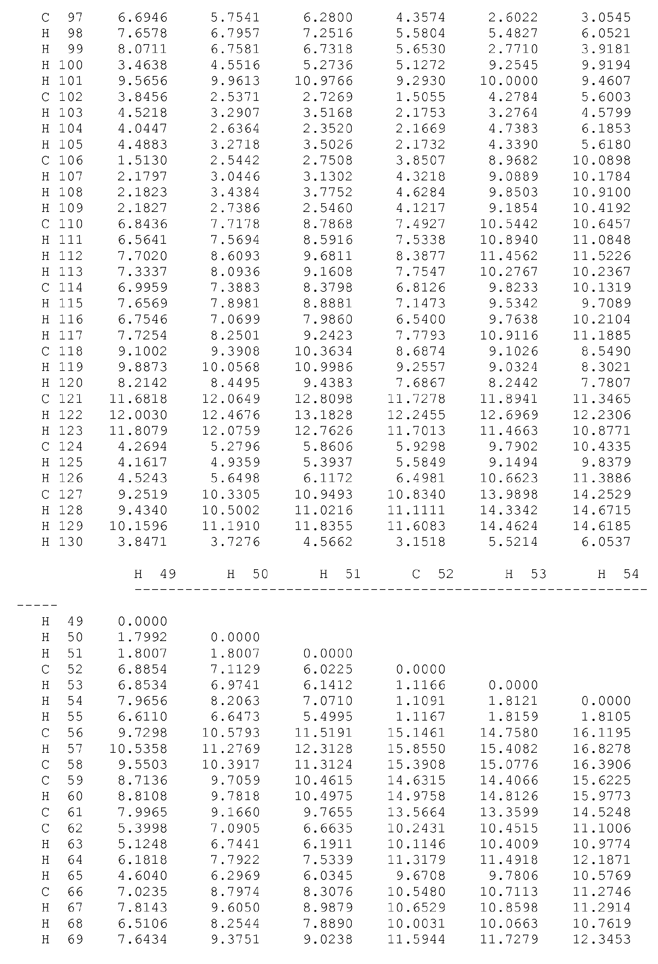

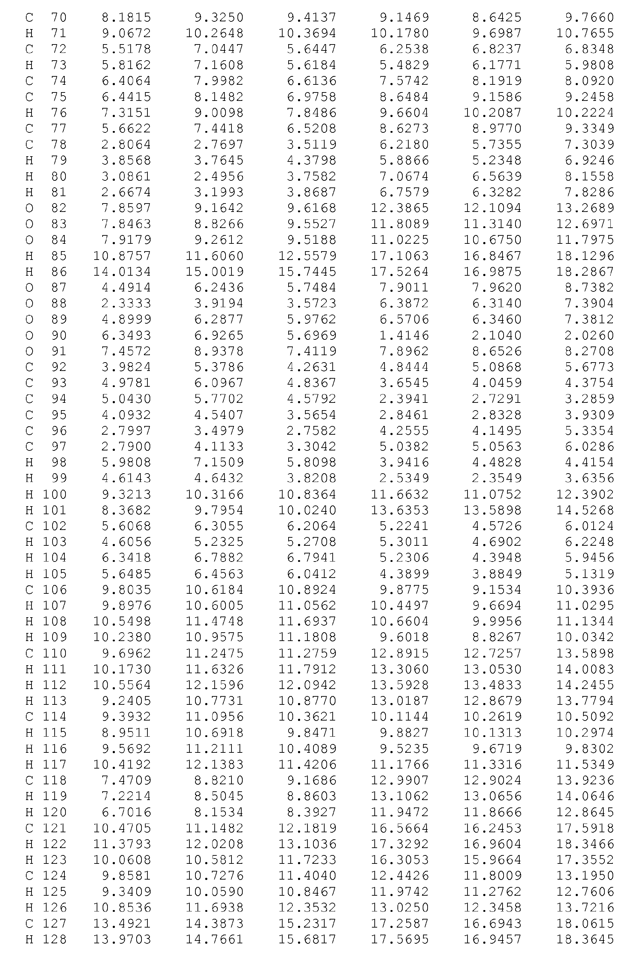

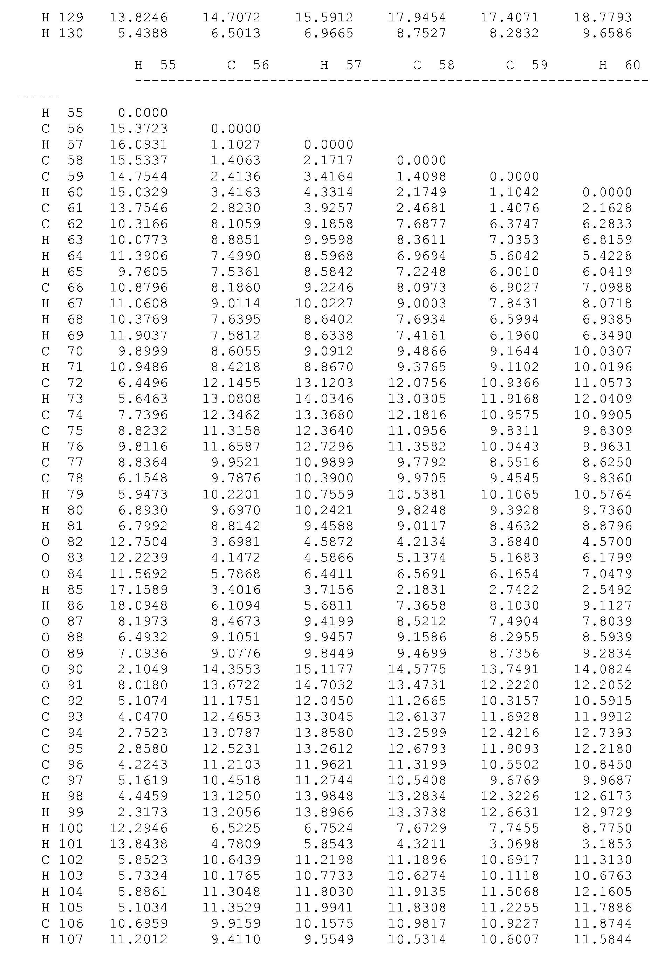

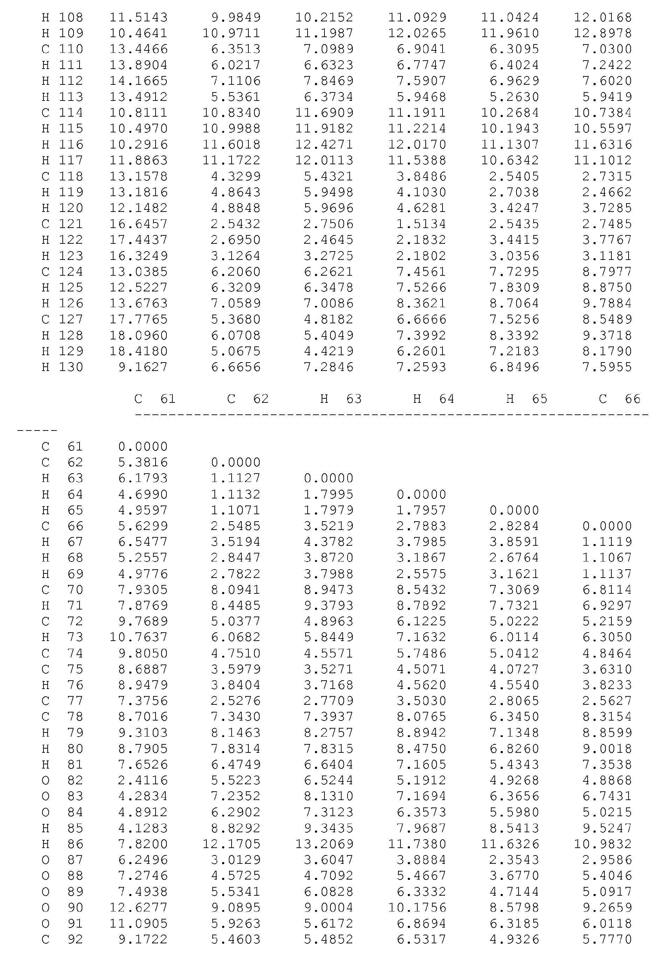

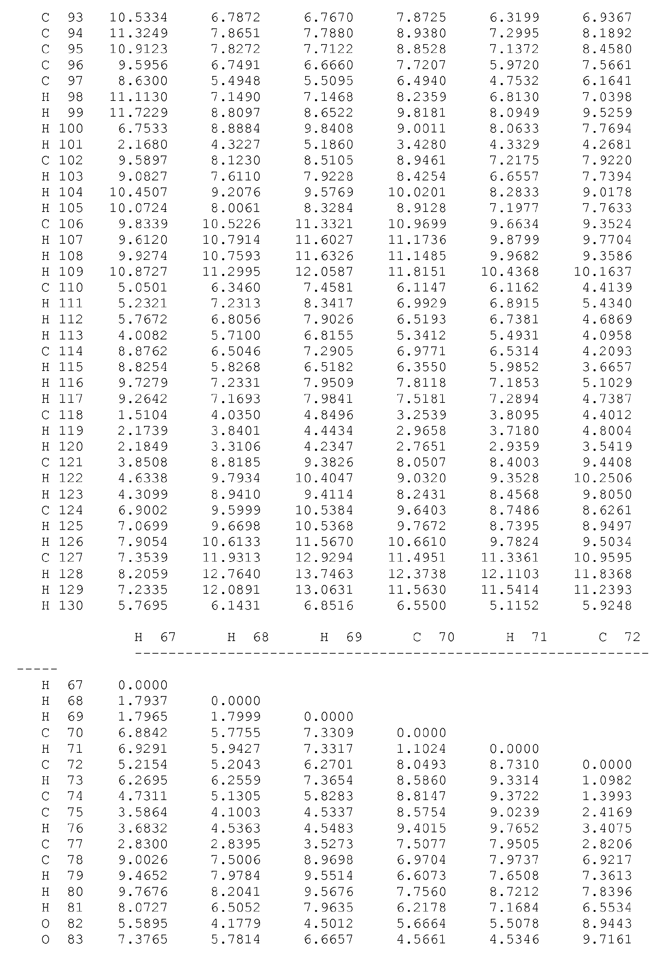

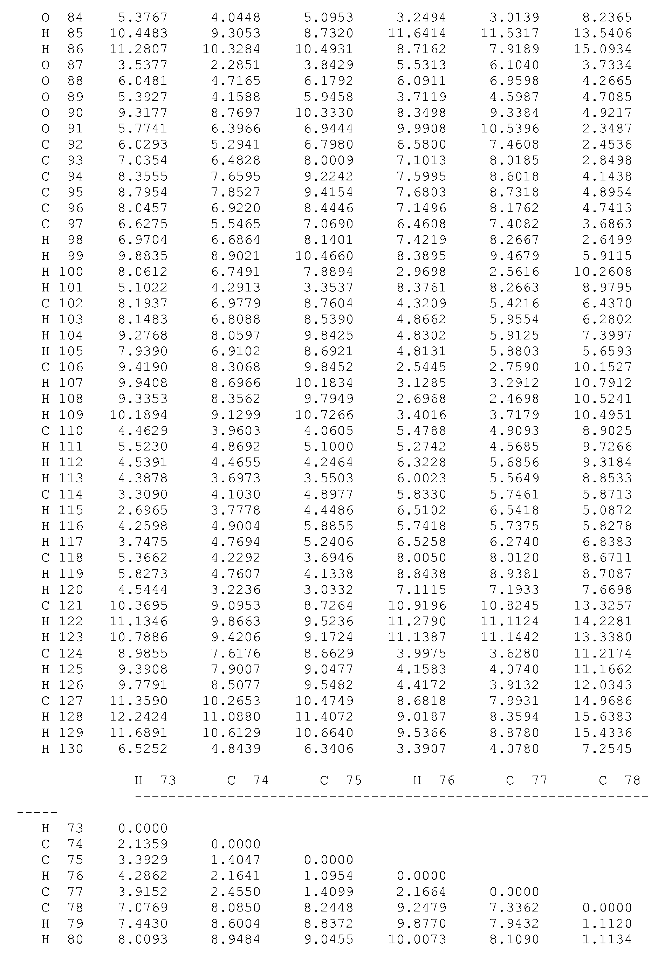

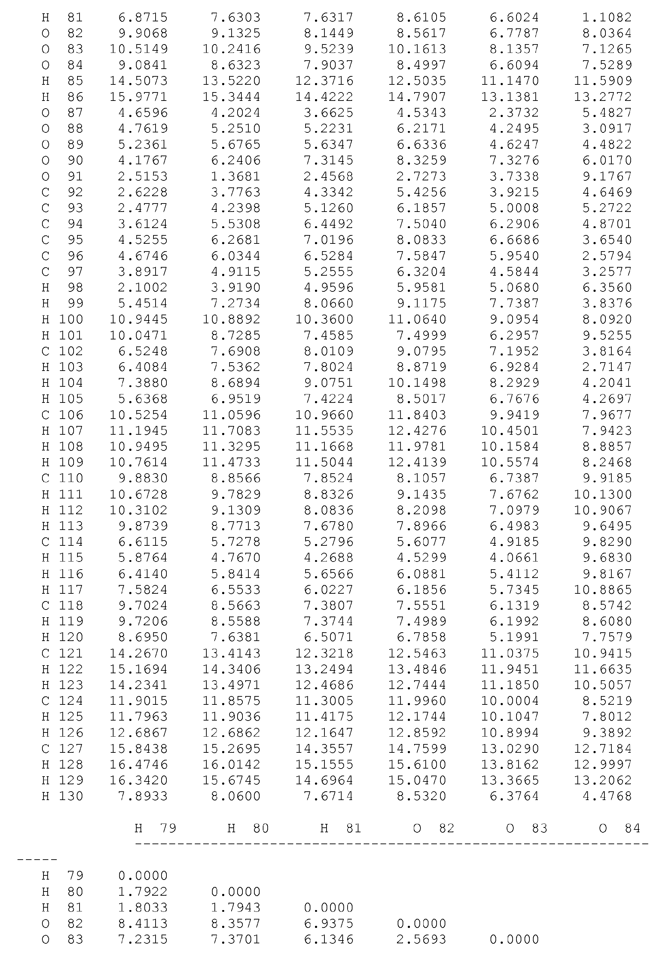

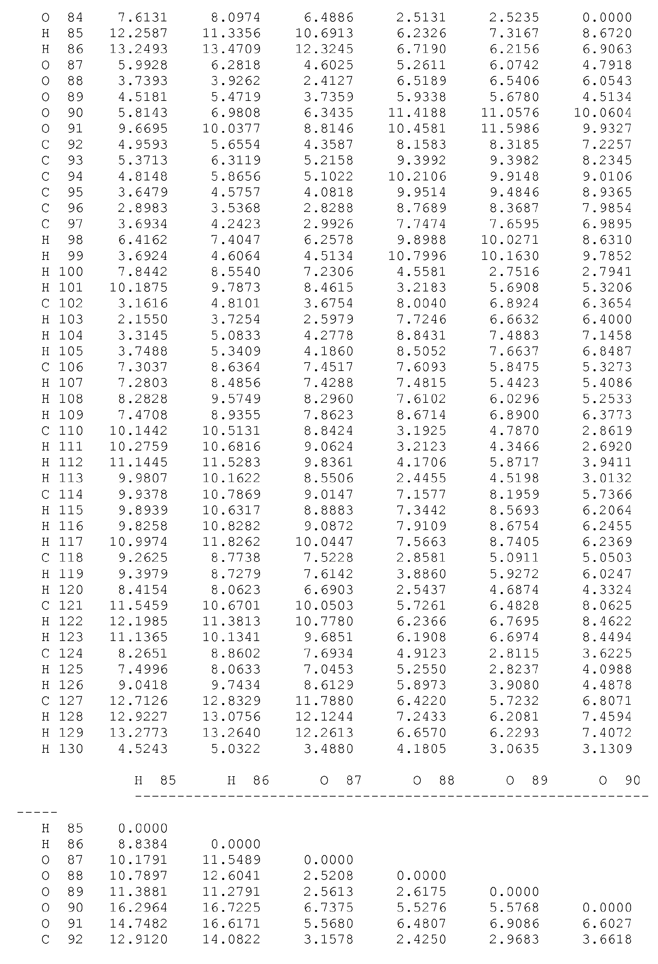

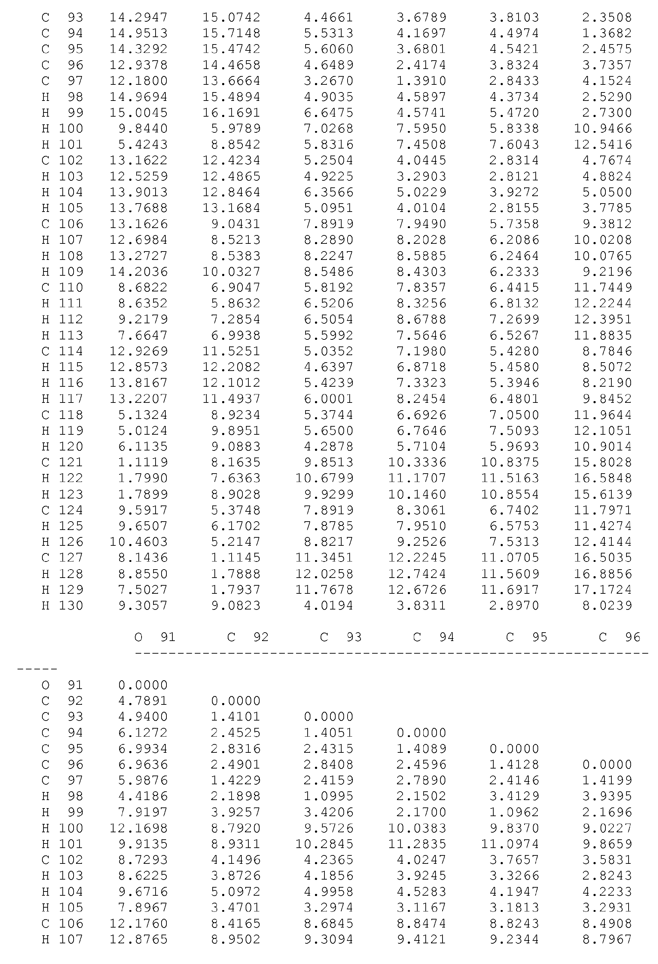

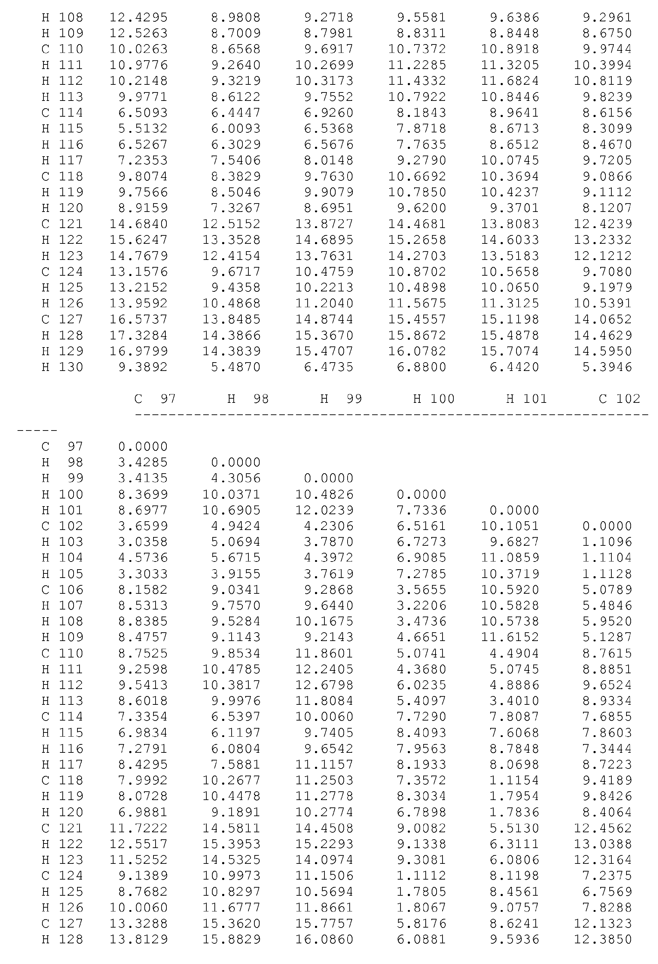

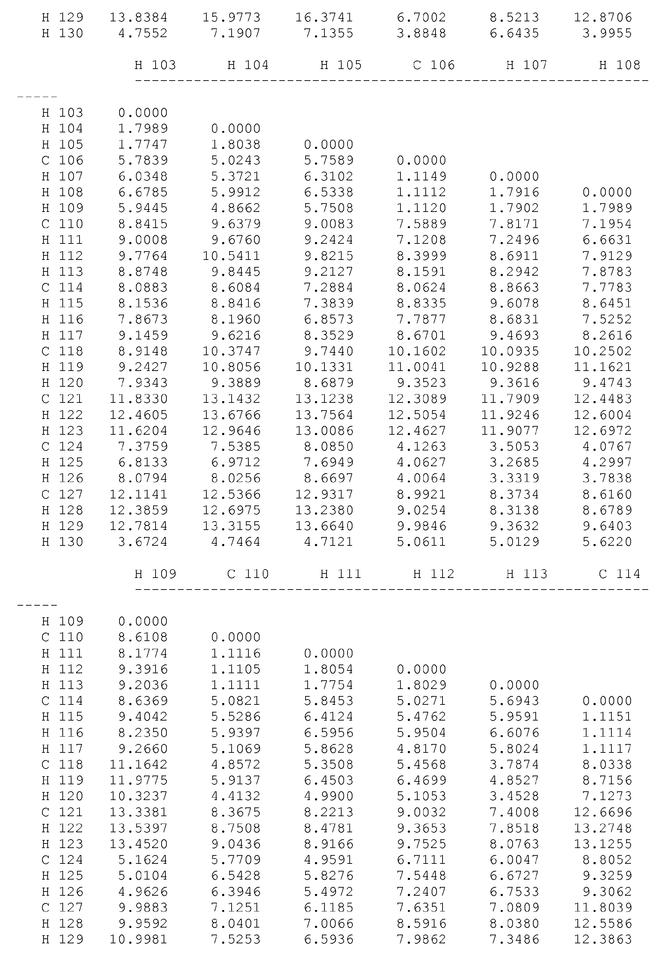

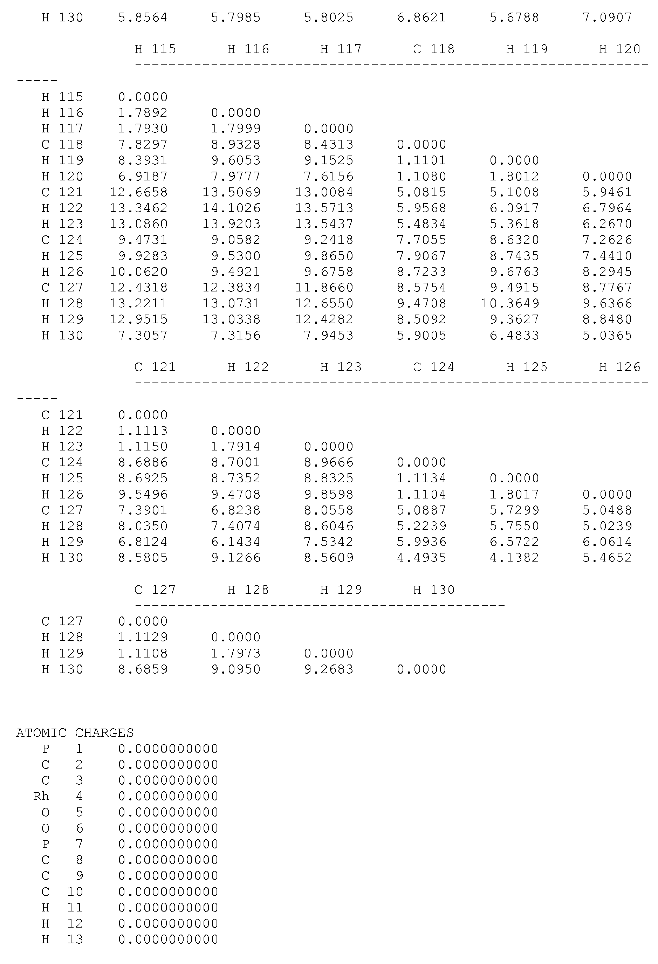

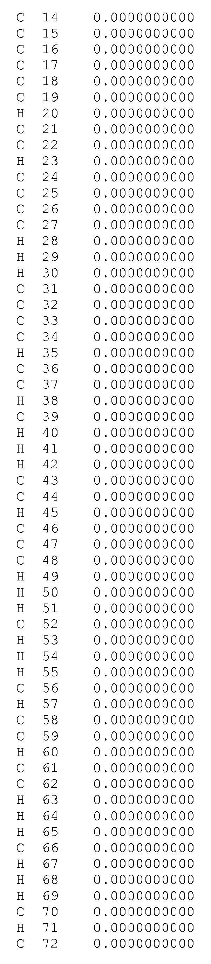

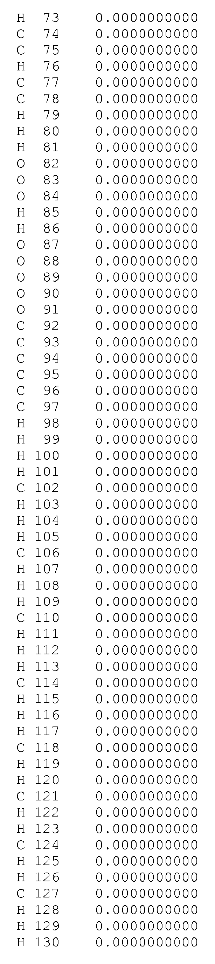

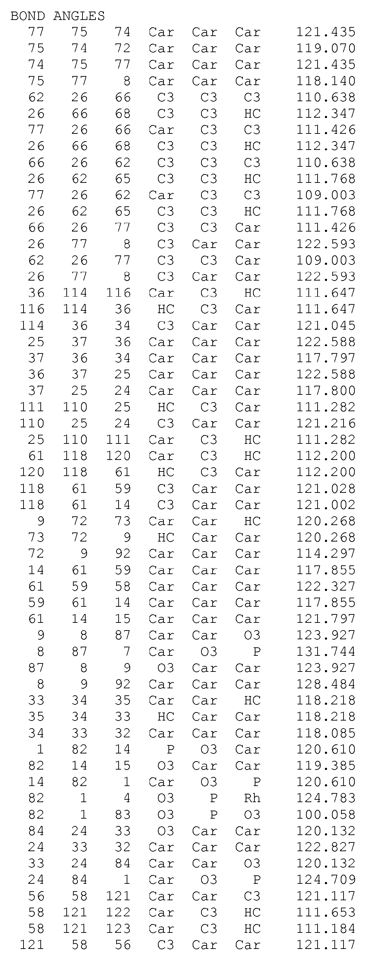

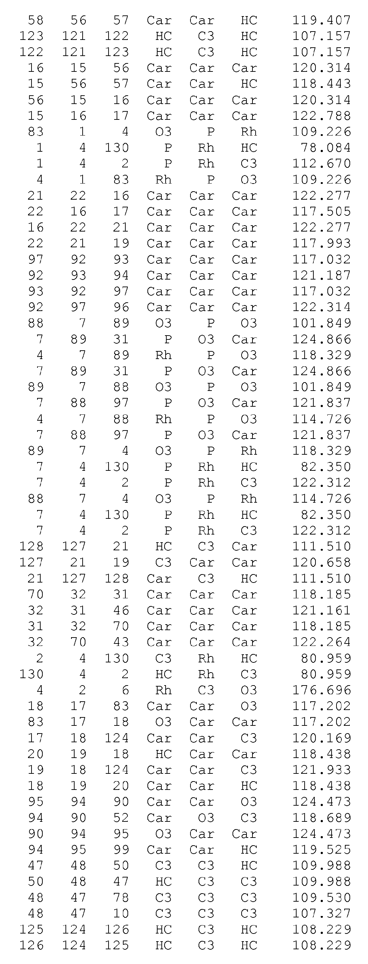

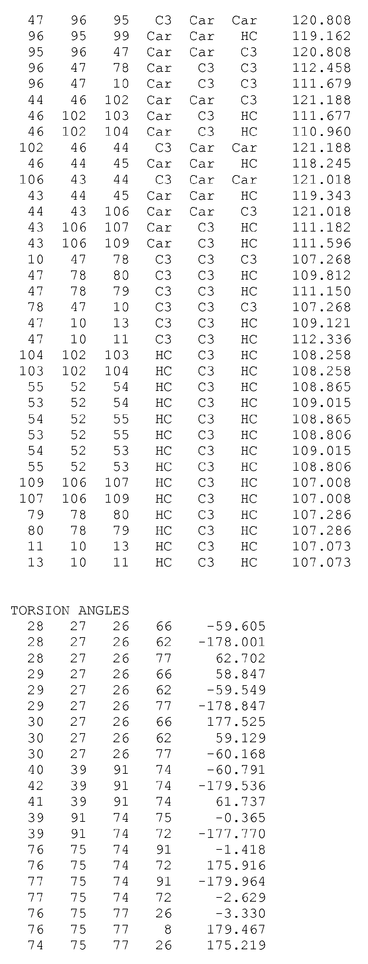

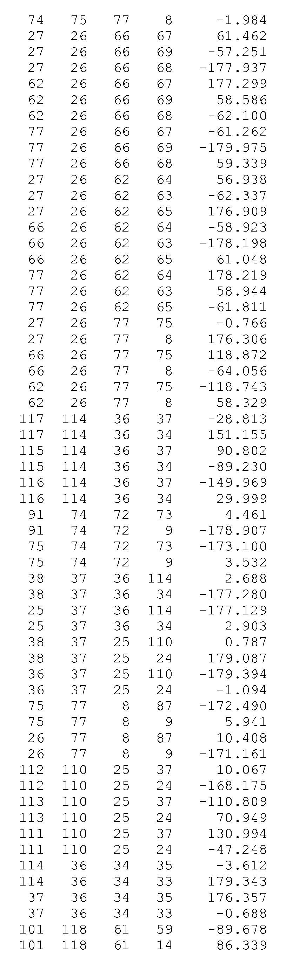

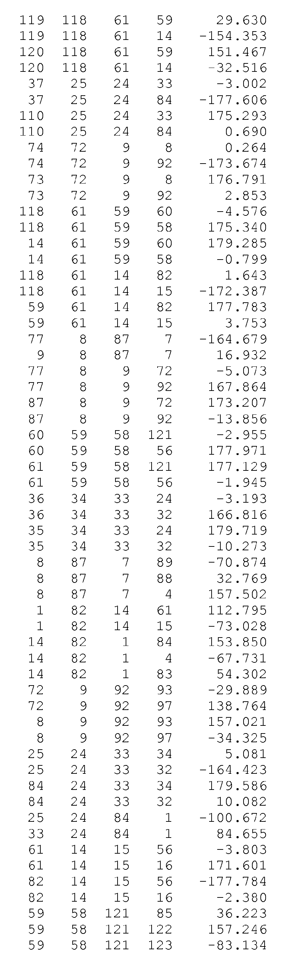

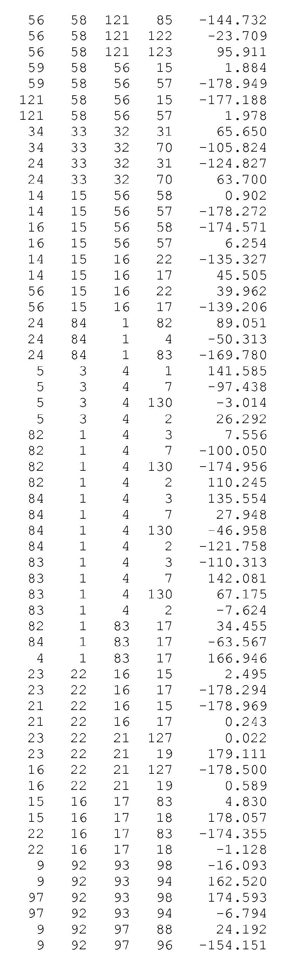

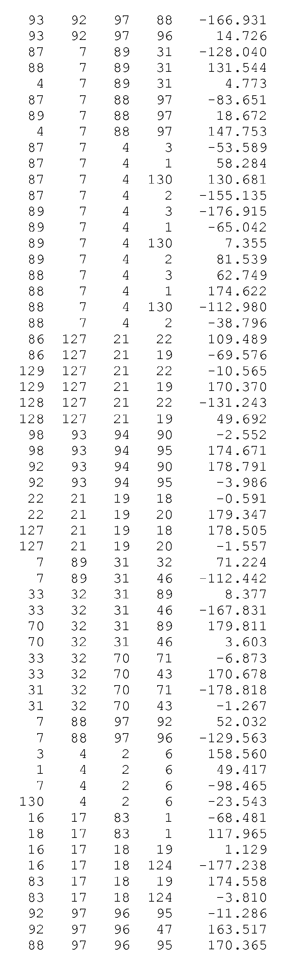

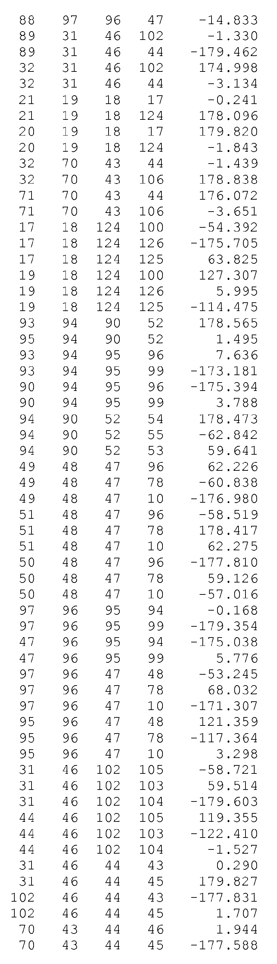

Figur 2 im Anhang liefert alle berechneten Koordinaten, Abstände und Winkel der Verbindung (3).

Die Figuren 3 und 4 zeigen Hydroformylierungsversuche in einer kontinuierlich betriebenen Versuchsanlage. In einer ersten Versuchsreihe (Figur 3) wurde die erfindungsgemäße Verbindung (1) getestet. Unter den gewählten Reaktionsbedingungen stellte sich eine Aldehydausbeute zwischen 80% und 90% ein. Dieser Zustand konnte bis zum Versuchsende konstant gehalten werden. Die prozentuale Verteilung zwischen n-Pentanal und 2-Methylbutanal, bzw. die Regio-Selektivität, betrug 92 % zu 8 %.

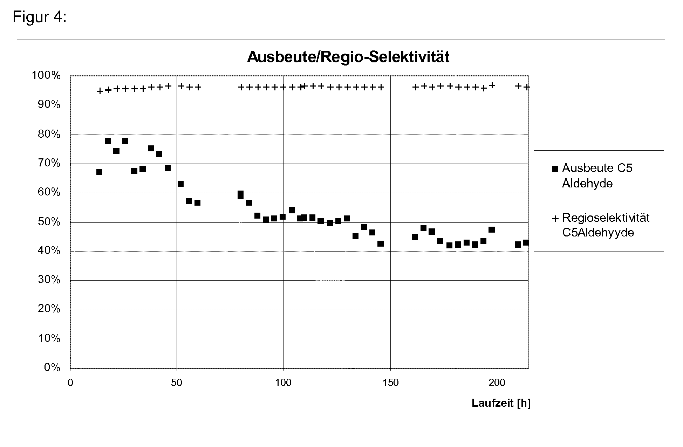

In der zweiten Versuchsreihe (Figur 4) wurde unter den gleichen Versuchsbedingungen die Vergleichsverbindung Biphephos verwendet. Unter den gewählten Reaktionsbedingungen fiel die Aldehydausbeute von anfänglich 70 % bis 80% nach 150 h auf 40% bis 50% ab. Die prozentuale Verteilung zwischen n-Pentanal und 2-Methylbutanal, bzw. die Regio-Selektivität, betrug 95 % zu 5 %. D.h. dieser Ligand zeichnet sich durch eine deutlich geringere Langzeitstabilität als der erfindungsgemäße Ligand (1) aus. Diese ist jedoch für einen großtechnischen Prozess entscheidend, da sie die Wirtschaftlichkeit eines solchen stark beeinflusst.

Folglich zeichnet sich der erfindungsgemäße Ligand durch eine deutlich verbesserte Stabilität und Aktivität aus. Dieses Ergebnis ist überraschend, da unsymmetrische substituierte Bisphosphite gegenüber symmetrisch substituierten deutlich an Aktivität und Selektivität einbüßen, wie bereits in Rhodium-catalyzed Hydroformylation, ed. by P.W.N.M. van Leeuwen et C. Claver, Kluwer Academic Publishers 2006, AA Dordrecht, NL, Seite 45-46 ausgeführt wird.

Der erfindungsgemäße Ligand (1) in der katalytisch aktiven Zusammensetzung zeichnet sich durch eine deutlich bessere Langzeitstabilität als die bisher im Stand der Technik beschriebenen Liganden aus und erfüllt somit die gestellte Aufgabe. Eine optimale Langzeitstabilität der katalytisch aktiven Zusammensetzung ist insbesondere in der großtechnischen Anwendung von Bedeutung, da der Ligand in der Hydroformylierungsreaktion großtechnisch zwar nachdosiert werden kann, jede Nachdosierung jedoch die Wirtschaftlichkeit eines großtechnischen Prozesses negativ beeinflusst und ihn ggf. unrentabel macht.

### Beispiele

### Allgemeine Reaktionsgleichung

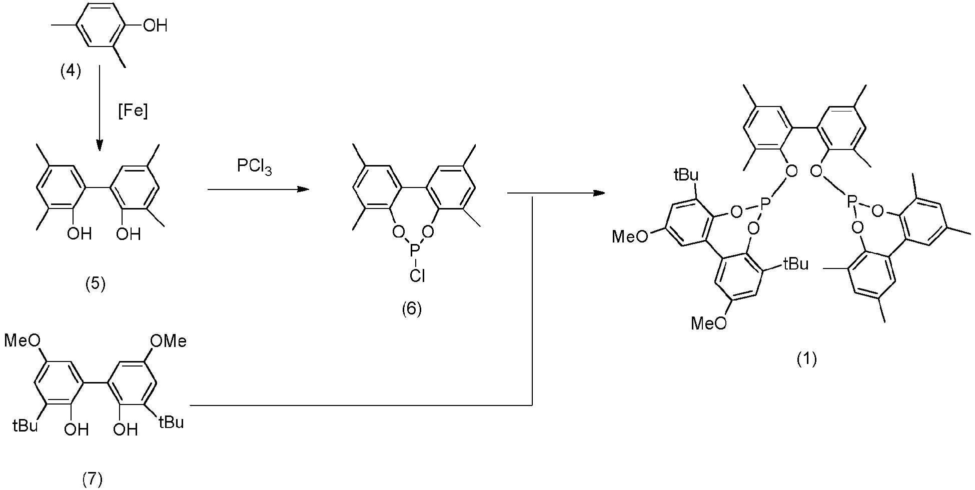

### Abkürzungen:

VE-Wasser = demineralisiertes Wasser
KPG = Kerngezogenes Präzisions-Glasgerät
ACN = Acetonitril
EtOAc = Ethylacetat
acac = acetylacetonat
NEt₃ = Triethylamin
TIPB = 1,2,4,5-Tetraisopropylbenzol

### Synthese des 2,2'-Bis(3,5-dimethylphenol) (5)

Das als Vorstufe eingesetzte Biphenol (5) wurde nach folgender Synthesevorschrift hergestellt.

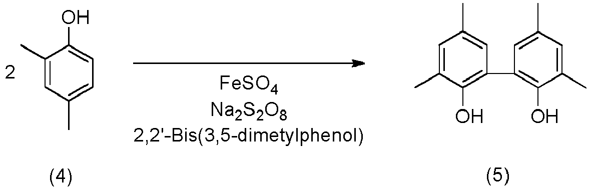

In einem 500 ml Schlenk mit KPG-Rührer, Zwischenaufsatz und Glasrührer wurden 1,42 g (0,005 mol) Eisen(II)-sulfatheptahydrat und 12,35 g (0,1 mol) 2,4-Dimethylphenol in 150 ml VE-Wasser und 5 ml Cyclohexan vorgelegt und auf 40 °C erwärmt.
In einem 100 ml Becherglas löste man 25.36 g (0,146 mol) Natriumperoxodisulfat in 80 ml VE-Wasser. Zum Start der Reaktion wurde eine kleine Portion Na₂S₂O₈-Lösung zum Phenol gegeben. Anschließend wurde alle 10 min eine kleinere Portion der Lösung hinzugegeben. Nach 30 min war die Na₂S₂O₈-Lösung Zugabe beendet.
Nach einer Reaktionszeit von 5h wurde zur Reaktionslösung 300 ml Cyclohexan und 200 ml Wasser hinzugegeben, 20 min rühren gelassen, dann warm in den Scheidetrichter überführt.
Die organische Phase wurde abgetrennt und bis zur Trockene eingeengt. Das Produkt (5) konnte in 69%iger Ausbeute (10,6 g) erhalten werden.

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).
Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84). Mittels der ³¹P-NMR wurde der Gehalt des Liganden (1) bestimmt, wobei dieser unsymmetrische Ligand durch zwei Phosphorsignale charakterisiert wird.

### Synthese des 2,2'-Bis-(3,5-dimethylphenol)chlorophosphits (6)

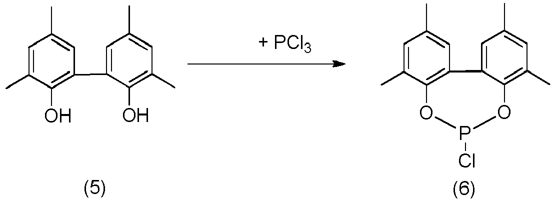

In einem sekurierten 2 L Schlenk mit Magnetrührer wurden 440 ml Phosphortrichlorid vorgelegt. In einem zweiten sekurierten 1 L Schlenk wurden 120 g 2,2' -Bis-(3,5-dimethylphenol) eingewogen und unter Rühren 500 ml getrocknetes Toluol hinzugefügt. Die Biphenol-Toluol-Suspension wurde innerhalb von 4 h bei 63°C zum Phosphortrichlorid dosiert. Nach vollständiger Zugabe wurde die Reaktionsmischung über Nacht bei Temperatur gerührt. Am nächsten Morgen wurde die Lösung in der Wärme (45°C) eingeengt und das Produkt konnten in 96,5%iger Ausbeute (153 g) erhalten werden. ³¹P-NMR: 175,59 (94,8% 2,2'-Bis-(3,5-dimethylphenol)chlorophosphit), 4,4% diverse PCI-Verbindungen, 0,8% P-H-Verbindung.

### Erfindungsgemäße Synthesevariationen zur Herstellung des reinen Liganden (1)

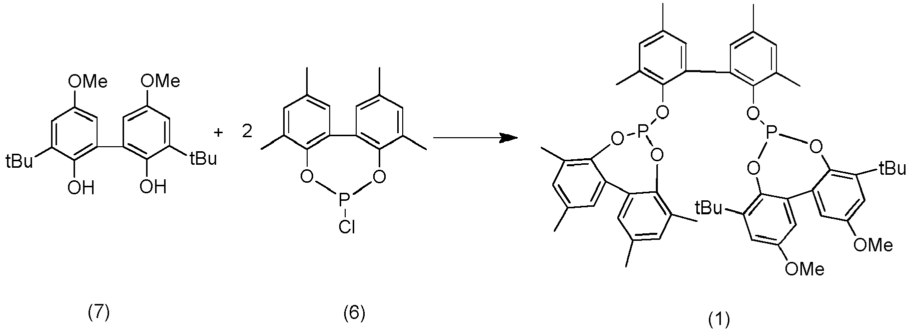

### Variante 1: ACN/NEt₃

In einem 1000 ml Schlenk wurde unter Schutzgas 38,75 g (0,121 mol) 2,2'-Bis-(3,5-dimethylphenyl)chlorophosphit in 150 ml entgastem ACN gelöst und auf 35°C erwärmt. In einem zweiten Schlenk (500 ml) wurden 20,1 g (0,056 mol) 3,3'-Di-tert.-butyl-5,5'-dimethoxy-[1,1'-biphenyl]-2,2'diol in 150 ml entgastem ACN gelöst und unter Rühren mit 40,9 ml entgastem Triethylamin (0,29 mol) versetzt. Dann wurde langsam die Biphenol/Trietylamin-Lösung zu der Chlorophosphitlösung getropft. Nach einer Nachreaktionszeit von1h wurde die Reaktionslösung über Nacht bei 45°C gerührt.
Dieser Feststoff wurde in entgastem ACN 1.5h bei 75°C suspendiert und abgetrennt und mit warmen ACN nachgewaschen. Anschließend wurde das Produkt in getr. Toluol 1.5h bei 35°C suspendiert und nachgewaschen. Das Zielprodukt (1) konnte als weißer Feststoff (33 g, 66%) erhalten werden. ³¹P-NMR (202,4 MHz, toluene-d₈): 142,5 und 140,9 (100%)

### Variante 2: EtOAc/NEt₃

In einem 100 ml Schlenk wurde unter Schutzgas 7,3 g (21,0 mmol) 2,2'-Bis-(3,5-dimethylphenyl)chlorophosphit in 15 ml entgastem Ethylacetat gelöst und auf 35°C erwärmt. In einem zweiten Schlenk (100 ml) wurden 3,9 g (9,5 mmol) 3,3'-Di-tert.-butyl-5,5'-dimethoxy-[1,1'-biphenyl]-2,2'diol in 7,0 ml NEt₃ gelöst. Anschließend wurde die Biphenol/Triethylamin-Lösung langsam innerhalb von 20 Minuten zu der Chlorophosphitlösung getropft. Die Lösung wurde eine weitere Stunde bei 35°C und anschließend über Nacht bei 45°C gerührt.
Dieser Feststoff wurde in entgastem ACN 1.5h bei 75°C suspendiert und abgetrennt und mit warmen ACN nachgewaschen. Anschließend wurde das Produkt in getr. Toluol 1.5h bei 35°C suspendiert und abgetrennt.
Das Zielprodukt (1) konnte als weißer Feststoff (5.0 g, 58%) erhalten werden. ³¹P-NMR (202,4 MHz, toluene-d₈): 142,5 und 140,9 (100%).

### Variante 3: EtOAc/Pyridin

In einem 250 ml Schlenk wurde unter Schutzgas 10,07 g (31,0 mmol) 2,2'-Bis-(3,5-dimethylphenyl)chlorophosphit in 20 ml entgastem Ethylacetat gelöst und auf 45°C erwärmt. In einem zweiten Schlenk (50 ml) wurden 5,54 g (15 mmol) 3,3'-Di-tert.-butyl-5,5'-dimethoxy-[1,1'-biphenyl]-2,2'diol in 26 ml Ethylacetat und 5,2 ml entgastem Pyridin gelöst. Anschließend wurde die Biphenol/Pyridin-Lösung langsam innerhalb von 30 Minuten zu der Chlorophosphitlösung getropft. Die Lösung wurde über Nacht bei 45°C gerührt.
Am nächsten Tag wurde die Lösung filtriert und der Feststoff mit ACN gewaschen. Das Zielprodukt konnte als weißer Feststoff (4,2g, 31%) erhalten werden. ³¹P-NMR (202,4 MHz, toluene-d₈): 142,2 und 141,1 (100%).

### Variante 4: Durchführung eines Tieftemperaturversuches bei -20°C

In einem 250 ml Schlenk wurde unter Schutzgas 8,0 g (0,025 mol) 2,2'-Bis-(3,5-dimethylphenyl)chlorophosphit in 30 ml entgastem ACN gelöst und auf -20°C gekühlt. In einem zweiten Schlenk (100 ml) wurden 4,32 g (0,012 mol) 3,3'-Di-tert.-butyl-5,5'-dimethoxy-[1,1'-biphenyl]-2,2'diol in 30 ml entgastem ACN gelöst und unter Rühren mit 8,5 ml entgastem Triethylamin versetzt. Dann wurde langsam die Biphenol-Triethylamin-Lösung bei -20°C zu der Chlorophosphitlösung getropft. Nach vollständiger Zugabe wurde für weitere 4 Stunden bei -20 °C weiter gerührt. Über Nacht wurde die Reaktionslösung bis zum Morgen bei -10 °C gerührt. Dieses Vorgehen, Reaktionstemperatur über Tag bei-20 °C und über Nacht bei -10 °C, wurde 3 Tage wiederholt durchgeführt. Im Anschluss daran wurde der Reaktionsansatz innerhalb von 3 Stunden auf RT gebracht. Anschließend wurde die Lösung filtriert und der Feststoff mit kaltem ACN gewaschen. Das Zielprodukt konnte als weißer Feststoff (7,6 g, 70%) erhalten werden. ³¹P-NMR (202,4 MHz, toluene-d₈): 142,5 und 140,9 (100%).
Das unsymmetrische Bisphosphit (1) konnte somit völlig überraschend und entgegen dem Stand der Technik auch bei tiefen Temperaturen in guten Ausbeuten und exzellenter Reinheit erhalten werden.

### Aufreinigung des Liganden (1):

Neben dem Suspendieren des Liganden in verschiedenen Lösungsmitteln (siehe obiges Beispiel) ist es auch möglich, den Liganden mittels Umkristallisation zu reinigen. Diese Umkristallisation erfolgte nach WO 2012095255. Anstelle o-Xylol kann auch Toluol zur Umkristallisation in analoger Weise verwendet werden.

### Arbeitsvorschrift für die Hydroformylierunasexperimente

### Versuchsbeschreibung - allgemein

Die Versuche wurden in 100 ml-Autoklaven der Fa. Parr Instrument durchgeführt. Die Autoklaven sind mit einer elektrischen Beheizung ausgerüstet. Die Druckkonstanthaltung erfolgt über Massedurchflußmesser und Druckregler. Während der Versuchszeit kann über eine Spritzenpumpe eine genau definierte Eduktmenge unter Reaktionsbedingungen eingespritzt werden. Über Kapillarleitungen und HPLC-Ventile können während der Versuchszeit Proben gezogen und sowohl über GC- als auch über LC-MS-Analytik untersucht werden.

### Versuchsbeschreibung - Langzeitversuch

Der Rh-Precursor (Rh(acac)(CO)₂) (acac= acetylacetonat) und der Ligand werden in 40 ml Isononylbenzoat im Autoklaven vorgelegt. Die Rh-Konzentration beträgt 100 ppm bezogen auf die gesamte eingesetzte Reaktionsmasse. Der Ligandüberschuß beträgt molar 4:1 bezogen auf Rhodium.
Als weitere Komponente wird im Verhältnis 2:1 zum Liganden die Verbindung **(Ib)** als Amin zugegeben. Als GC-Standard werden 0,5 g 1,2,4,5-Tetraisopropylbenzol hinzugegeben.
Reaktionstemperatur ist 120 °C. Der Reaktionsdruck beträgt 20 bar Synthesegas (H₂:CO=50:50 Vol%).

Als Olefin wurden mit der Spritzenpumpe in Abständen von ca. 1 Tag jeweils 4 ml cis-2-Buten zudosiert. GC-Proben wurden nach 1, 2, 4 Stunden und vor der nächsten Dosierung gezogen.

Es wurden folgende Liganden hinsichtlich ihrer Stabilität untersucht:

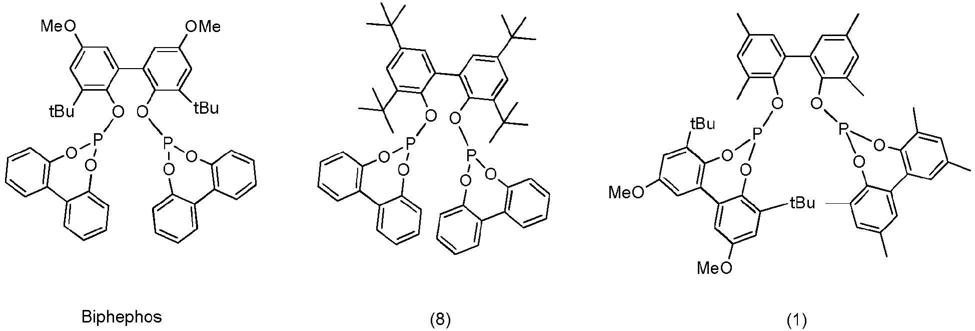

### Ergebnisse - Langzeitversuche

Die relativen Aktivitäten werden durch das Verhältnis von k 1.Ordnung zu k0, d.h. dem k-Wert zum Zeitpunkt 0 der Reaktion (Reaktionsstart), bestimmt und beschreiben die relative Aktivitätsabnahme während der Versuchslaufzeit.
Die k-Werte 1.Ordnung erhält man aus einer Auftragung von (-In(1-Umsatz)) gegen die Zeit.

**Tabelle 2:**

| | | **Dosierung bei** | **k 1. Ordnung** | **k/k0** | |
|---|---|---|---|---|---|
| **Lfd. Nr.** | **Ligand** | **Laufzeit (h)** | **(min^-1)** | **rel. Aktivität** | **n/i-Selektivität** |
| 1 | Biphephos | 0 | 1,39E-02 | 1 | 21 |
| 2 | Biphephos | 20,5 | 4,45E-03 | 0,32 | 21 |
| 3 | Biphephos | 44,3 | 2,91E-03 | 0,209 | 20 |
| 4 | Biphephos | 66,6 | 1,72E-03 | 0,124 | 20 |
| 5* | Ligand (1) | 0 | 7,74E-03 | 1 | 17 |
| 6* | Ligand (1) | 20,8 | 5,10E-03 | 0,659 | 16 |
| 7* | Ligand (1) | 44,8 | 3,19E-03 | 0,412 | 15 |
| 8* | Ligand (1) | 117,8 | 2,99E-03 | 0,386 | 14 |
| 9 | Ligand (8) | 0 | 1,72E-02 | 1 | 14 |
| 10 | Ligand (8) | 22,4 | 9,00E-03 | 0,523 | 13 |
| 11 | Ligand (8) | 44,7 | 5,39E-03 | 0,313 | 13 |
| 12 | Ligand (8) | 68,3 | 3,31E-03 | 0,192 | 13 |

| | | | | | |
|---|---|---|---|---|---|
| * erfindungsgemäß | | | | | |

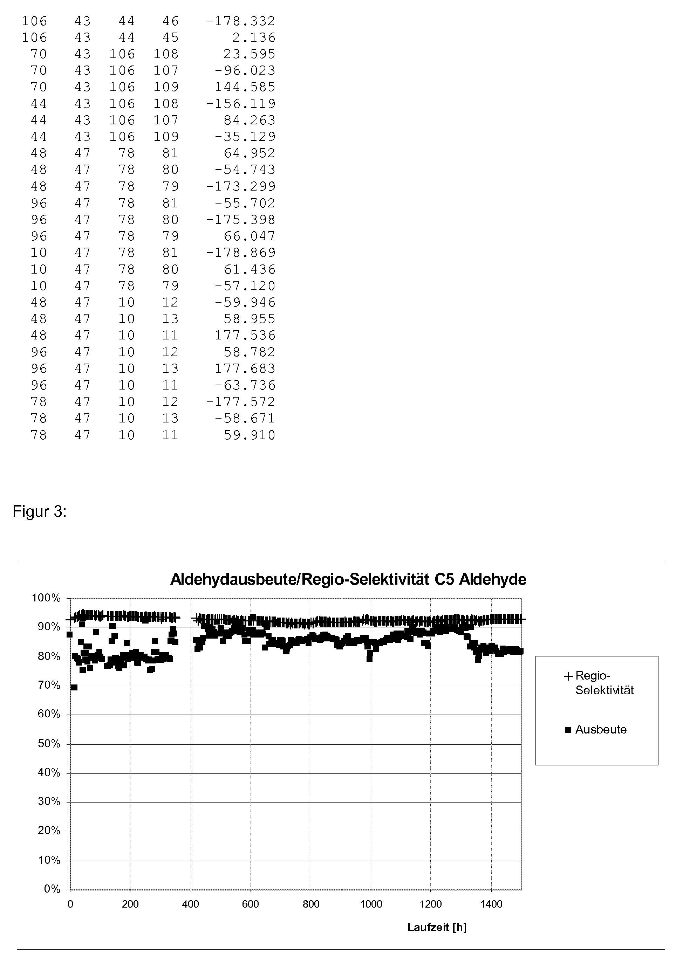

Resultat: Der Aktivitätsabfall des Katalysators mit den Liganden Biphephos und (8) ist (Tabelle 2; Einträge 1-4, 9-12) deutlich stärker als mit dem Liganden (1). Bemerkenswert ist, dass die relative Aktivität des Liganden (1) nach annähernd der doppelten Reaktionszeit (Tabelle 2; Eintrag 8) immer noch mehr als doppelt so hoch ist wie bei den anderen beiden Liganden nach der halben Reaktionszeit (Tabelle 2; Einträge 4 und 12) bei weiterhin sehr guten n/i-Selektivitäten. Dieses Verhalten wird in den Langzeitversuchen in der kontinuierlich betriebenen Hydroformylierungsanlage (siehe Figuren 3 und 4) bestätigt. Es konnte somit ein unsymmetrischer Ligand hergestellt und in einer hydroformylierungsaktiven Zusammensetzung verwendet werden, der völlig überraschend und entgegen dem Stand der Technik sehr gute Eigenschaften aufweist und die technische Aufgabe löst.

### Erfindungsgemäße Ergebnisse - Substratvariation

### Beispiel 1

In einem 100 ml-Autoklaven der Fa. Parr Instruments wurde bei 120 °C und 30 bar 5.3 g Propen hydroformyliert. Als Precursor wurden 0.0054 g Rh(acac)(CO)₂ in 43.89 g Toluol vorgelegt. Als Ligand wurden 0.0701 g Ligand (1) in der Katalysatoransatzlösung eingesetzt. Als organisches Amin wurden 0.0372 g der Verbindung (Ib) sowie 0.5016 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen. Es wurden 89.6 mol-% Butanal, 7.9 mol-% 2-Methylpropanal und 2,3 mol-% Propan gebildet. Die Regioselektivität zu n-Butanal beträgt 92.0 %.

### Beispiel 2

In einem 100 ml-Autoklaven der Fa. Parr Instruments wurde bei 120 °C und 20 bar 5.6 g cis-2-Buten hydroformyliert. Als Precursor wurden 0.0056 g Rh(acac)(CO)₂ in 48.8 g Toluol vorgelegt. Als Ligand wurden 0.0779 g Ligand (1) in der Katalysatoransatzlösung eingesetzt. Als organisches Amin wurden 0.0416 g der **Verbindung (Ib)** sowie 0.5760 g TIPB als GC-Standard zugefügt. Das Edukt wurde nach Erreichen der vorgesehenen Reaktionstemperatur zudosiert.
Während der Reaktion wurde der Druck über eine Synthesegasregelung mit Massedurchflussmesser konstant gehalten. Proben wurden aus der Reaktionsmischung nach 20 Stunden gezogen. Es wurden 80.0 mol-% Pentanal, 5.2 mol-% 2-Methylbutanal und 3.7 mol-% n-Butan gebildet. Die Regioselektivität zu n-Pentanal beträgt 94.0 %.

### Beispiel 3

In einem 100 ml-Autoklaven der Fa. Parr Instruments wurde bei 120 °C und 20 bar 6.3 g Isobuten hydroformyliert. Als Precursor wurden 0.0046 g Rh(acac)(CO)₂ in 39.8 g Toluol vorgelegt. Als Ligand wurden 0.0636 g Ligand (1) in der Katalysatoransatzlösung eingesetzt. Als organisches Amin wurden 0.0339 g der Verbindung (Ib) und 0.4701 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen. Es wurden 72.9 mol-% 3-Methylbutanal, 0.1 mol-% Pivalinaldehyd und 4.4 mol-% iso-Butan gebildet.

### Beispiel 4

In einem 100 ml-Autoklaven der Fa. Parr Instruments wurde bei 120 °C und 20 bar 6.7 g eines C-4-Gemisches mit folgender Zusammensetzung: 2.9 mol-% Isobutan, 9,9 mol-% n-Butan, 28.7 mol-% 1-Buten, 43.5 mol-% Isobuten, 14,6 mol % 2-Butene und 0.2 mol % 1,3-Butadien hydroformyliert. Als Precursor wurden 0.0049 g Rh(acac)(CO)₂ in 42.38 g Toluol vorgelegt. Als Ligand wurden 0.0697 g Ligand (1) in der Katalysatoransatzlösung eingesetzt. Als organisches Amin wurden 0.0374 g der Verbindung (Ib) sowie 0.5069 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen. Der Austrag enthält 32.86 % 3-Methylbutanal (Umsatz Isobuten 75.6 mol -%), 39.0 mol-% n-Pentanal und 1.8 mol-% 2-Methylbutanal (Umsatz Butene 76.5 mol-%, Regioselektivität zu n-Pentanal 95.6 %). Als Hydrierprodukte werden im Austrag 4.7 mol-% Isobutan und 11.3 mol % n-Butan gefunden.

### Beispiel 5

In einem 100 ml-Autoklaven der Fa. Parr Instruments wurde bei 120 °C und 20 bar 6.5 g ein C-4-Gemisch mit folgender Zusammensetzung: 5.9 mol-% Isobutan, 15.6 mol-% n-Butan, 52.9 mol-% 1-Buten, 0.1 mol-% Isobuten, 24.8 mol % 2-Butene und 0.5 mol % 1,3-Butadien hydroformyliert. Als Precursor wurden 0.0052 g Rh(acac)(CO)₂ in 45.05 g Toluol vorgelegt. Als Ligand wurden 0.0727 g Ligand (1) in der Katalysatoransatzlösung eingesetzt. Als organisches Amin wurden 0.0377 g der Verbindung (Ib) sowie 0.5314 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen. Der Austrag enthält 0.14 mol-% 3-Methylbutanal, 69.5 mol-% n-Pentanal und 3.67 mol-% 2-Methylbutanal (Umsatz Butene 94.2 mol-%, Regioselektivität zu n-Pentanal 96.5 %). Als Hydrierprodukte werden im Austrag 5.64 mol-% Isobutan und 18.55 mol % n-Butan gefunden.

### Beispiel 6

In einem 100 ml-Autoklaven der Fa. Parr Instruments wurde bei 120 °C und 20 bar 7.0 g eines C-4-Gemisches mit folgender Zusammensetzung: Das Edukt enthält 5.9 mol-% Isobutan, 22.1 mol-% n-Butan, 45.5 mol-% 1-Buten, 2.1 mol-% Isobuten, 17.1 mol % 2-Butene und 0.2 mol % 1,3-Butadien hydroformyliert. Als Precursor wurden 0.0047 g Rh(acac)(CO)₂ in 40.81 g Toluol vorgelegt. Als Ligand wurden 0.0659 g Ligand (1) in der Katalysatoransatzlösung eingesetzt. Als organisches Amin wurden 0.0342 g der Verbindung (Ib) und 0.4814 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen. Der Austrag enthält 1.5 mol-% 3-Methylbutanal (Umsatz Isobuten 71.6 mol-%), 61.9 mol-% n-Pentanal und 2.9 mol-% 2-Methylbutanal (Umsatz Butene 93.3 mol-%, Regioselektivität zu n-Pentanal 95.5 %). Als Hydrierprodukte werden im Austrag 5.3 mol-% Isobutan und 23.4 mol % n-Butan gefunden.

### Beispiel 7

In einem 100 ml-Autoklaven der Fa. Parr Instruments wurde bei 120 °C und 20 bar 7.1 g eines C-4-Gemisches mit folgender Zusammensatzung: 3.5 mol-% Isobutan, 13.0 mol-% n-Butan, 47.3 mol-% 1-Buten, 13.9 mol-% Isobuten, 21.6 mol % 2-Butene und 0.4 mol % 1,3-Butadien hydroformyliert. Als Precursor wurden 0.0048 g Rh(acac)(CO)₂ in 43.88 g Toluol vorgelegt. Als Ligand wurden 0.0680 g Ligand (1) in der Katalysatoransatzlösung eingesetzt. Als organisches Amin wurden 0.0363 g der Verbindung (Ib) und 0.5092 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen. Der Austrag enthält 10.1 mol-% 3-Methylbutanal (Umsatz Isobuten 72.8 mol-%), 63.2 mol-% n-Pentanal und 3.2 mol-% 2-Methylbutanal (Umsatz Butene 96.3 mol-%, Regioselektivität zu n-Pentanal 95.2 %). Als Hydrierprodukte werden im Austrag 3.5 mol-% Isobutan und 15.1 mol % n-Butan gefunden.

### Beispiel 8

In einem 100 ml-Autoklaven der Fa. Parr Instruments wurde bei 120 °C und 20 bar 5.8 g eines C-4-Gemisches mit folgender Zusammensetzung: 0.1 mol-% Isobutan, 27.6 mol-% n-Butan, 27.9 mol-% 1-Buten, 0.1 mol-% Isobuten und 44.0 mol % 2-Butene hydroformyliert. Als Precursor wurden 0.0051 g Rh(acac)(CO)₂ in 43.77 g Toluol vorgelegt. Als Ligand wurden 0.0699 g Ligand (1) in der Katalysatoransatzlösung eingesetzt. Als organisches Amin wurden 0.0373 g der Verbindung (Ib) und 0.5166 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen. Der Austrag enthält 59.9 mol-% n-Pentanal und 3.3 mol-% 2-Methylbutanal (Umsatz Butene 91.7 mol-%, Regioselektivität zu n-Pentanal 94.7 %). Als Hydrierprodukte werden im Austrag 0.1 mol-% Isobutan und 31.7 mol % n-Butan gefunden.

### Beispiel 9

In einem 100 ml-Autoklaven der Fa. Parr Instruments wurde bei 120 °C und 20 bar 6.0 g eines C-4-Gemisches mit folgender Zusammensetzung: 63.6 mol-% n-Butan, 1.0 mol-% 1-Buten und 35.8 mol % 2-Butene hydroformyliert. Als Precursor wurden 0.0041 g Rh(acac)(CO)₂ in 35.88 g Toluol vorgelegt. Als Ligand wurden 0.0573 g Ligand (1) in der Katalysatoransatzlösung eingesetzt. Als organisches Amin wurden 0.0306 g der Verbindung (Ib) und 0.4235 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen. Der Austrag enthält 29.7 mol-% n-Pentanal und 1.9 mol-% 2-Methylbutanal (Umsatz Butene 85.3 mol-%, Regioselektivität zu n-Pentanal 94.0 %).

### Beispiel 10

In einem 100 ml-Autoklaven der Fa. Parr Instruments wurde bei 120 °C und 20 bar 5.0 g n-Octene hydroformyliert. Als Precursor wurden 0.0049 g Rh(acac)(CO)₂ in 41.29 g Toluol vorgelegt. Als Ligand wurden 0.0669 g Ligand (1) in der Katalysatoransatzlösung eingesetzt. Als organisches Amin wurden 0.0378 g der Verbindung (Ib) und 0.5030 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen. Der Austrag enthält 54.2 mol-% Aldehyde (Regioselektivität zu n-Nonanal 90.9 %). Als Hydrierprodukte werden im Austrag 3.9 mol % n-Octan und 3.2 % Nonanol gefunden

### Beispiel 11

In einem 100 ml-Autoklaven der Fa. Parr Instruments wurde bei 120 °C und 20 bar 7.0 g 1,3-Butadien hydroformyliert. Als Precursor wurden 0.0054 g Rh(acac)(CO)₂ in 46.82 g Toluol vorgelegt. Als Ligand wurden 0.0770 g Ligand (1) in der Katalysatoransatzlösung eingesetzt. Als organisches Amin wurden 0.0413 g der Verbindung (Ib) und 0.5599 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen. Der Austrag enthält 0.2 mol-% n-Butan, 11.3 % n-Butene, 12.9 % Aldehyde und 11.5 mol-% 4-Vinyl-Cyclohexen. Der Gesamtumsatz an 1-3-Butadien beträgt 37.2 %.

### Beispiel 12

In einem 100 ml-Autoklaven der Fa. Parr Instruments wurde bei 120 °C und 20 bar 5.6 g Ölsäuremethylester hydroformyliert. Als Precursor wurden 0.0052 g Rh(acac)(CO)₂ in 44.06 g Toluol vorgelegt. Als Ligand wurden 0.0689 g Ligand (1) in der Katalysatoransatzlösung eingesetzt. Als organisches Amin wurden 0.0375 g der Verbindung (Ib) und 0.5260 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen. Aus ¹H- und ¹³C-NMR-Spektren wurden eine Aldehydausbeute von 49.5 mol-% berechnet. Die Regioselektivität zu endständigen Aldehyde beträgt 20.6 mol-%. Der Doppelbindungsanteil beträgt 35.9 mol-%.

### Beispiel 13

In einem 100 ml-Autoklaven der Fa. Parr Instruments wurde bei 120 °C und 20 bar 6.9 g eines Kohlenwasserstoffgemisches aus katalytisch betriebenen Spaltanlagen mit folgender Zusammensetzung: 1.5 mol-% Propan, 0.8 mol-% Propen, 28.1 mol-% Isobutan, 8.1 mol-% n-Butan, 16.4 mol-% 1-Buten, 16.9 mol-% Isobuten, 28.2 mol-% 2-Butene, 0.5 mol-% 1,3-Butadien und Anteile an C5-Olefinen und -Kohlenwasserstoffen hydroformyliert. Als Precursor wurden 0.0048 g Rh(acac)(CO)₂ in 43.39 g Toluol vorgelegt. Als Ligand wurden 0.0672 g Ligand (1) in der Katalysatoransatzlösung eingesetzt. Als organisches Amin wurden 0.0359 g der Verbindung (Ib) und 0.5035 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen.
Der Austrag enthält 1.3 mol-% Propan, 0.7 mol-% Butanal, 27.5 mol-% Isobutan, 9.6 mol-% n-Butan, 13.1 mol-% 3-Methylbutanal (77.4 % Isobutenumsatz), 39.1 mol-% Pentanal, 2.1 mol % 2-Methylbutanal (Umsatz n-Butene 96.9 %, Regioselektivtät zu n-Pentanal 95.0 %).

### Beispiel 14

In einem 100 ml-Autoklaven der Fa. Parr Instruments wurde bei 120 °C und 50 bar 1.8 g Ethen hydroformyliert. Als Precursor wurden 0.0050 g Rh(acac)(CO)₂ in 42.68 g Toluol vorgelegt. Als Ligand wurden 0.0668 g Ligand (1) in der Katalysatoransatzlösung eingesetzt. Als organisches Amin wurden 0.0363 g der Verbindung (Ib) und 0.5095 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen. Der Umsatz zu Propanal beträgt 98.7 %.

### Vergleichsversuch - Unsymmetrische und symmetrische Liganden

Neben der Testung des erfindungsgemäßen unsymmetrischen Liganden (1) mit verschiedenen Substraten wurden weiterhin symmetrische Liganden und ihr entsprechendes unsymmetrisches Isomer unter vergleichbaren Bedingungen getestet.
Zunächst wurde der im Stand der Technik bereits erwähnte symmetrische Biphephosligand und sein unsymmetrisches Isomer (9) getestet. Die Verbindung (9) wurde analog der Synthesevorschrift in WO 95/28228 auf Seite 19 hergestellt.

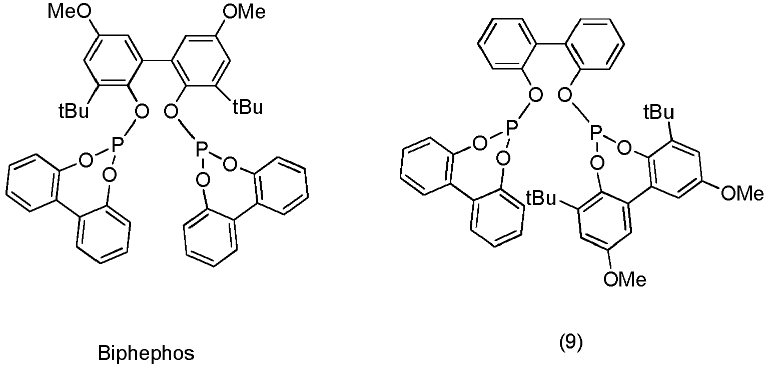

Die Versuche wurden nach der nachfolgenden Vorschrift durchgeführt:

### Beispiel 15

In einem 100 ml-Autoklaven der Fa. Parr Instruments wurde bei 120 °C und 20 bar 5.7 g cis-2-Buten hydroformyliert. Als Precursor wurden 0.0054 g Rh(acac)(CO)₂ in 51.5 g Diphyl (Gemisch aus ca. 73,5% Diphenyloxid und 26,5% Diphenyl) vorgelegt. Als Ligand wurden 0.0779 g des entsprechenden Liganden in der Katalysatoransatzlösung eingesetzt. Als organisches Amin wurden 0.0416 g der Verbindung (Ib) sowie 0.5760 g TIPB als GC-Standard zugefügt. Das Edukt wurde nach Erreichen der vorgesehenen Reaktionstemperatur zudosiert.
Während der Reaktion wurde der Druck über eine Synthesegasregelung mit Massedurchflussmesser konstant gehalten. Proben wurden aus der Reaktionsmischung nach 12 Stunden gezogen.

Die Ergebnisse sind in der Tabelle 3 dargestellt.

**Tabelle 3:**

| Eintrag | Ligand | Ausbeute Aldehyde | Regioselektivität n- Pentanal in % |
|---|---|---|---|
| 1 | Biphephos | 95,0 | 94,5 |
| 2 | (9) | 66,6 | 78,5 |

Das unsymmetrische Isomer (Ligand 9; Eintrag 2) des symmetrischen Biphephos zeichnet sich also durch eine deutlich geringere Aktivität sowie durch eine viel schlechtere Selektivität als der symmetrische Biphephosligand aus. Dies entspricht dem Stand der Technik. Die Verwendung beider Liganden, also des symmetrischen Biphephosliganden und seines unsymmetrischen Isomers ist bereits in Rhodium-catalyzed Hydroformylation, ed. by P.W.N.M. van Leeuwen et C. Claver, Kluwer Academic Publishers 2006, AA Dordrecht, NL beschrieben. Auf Seite 45-46, Tabelle 2 sind die Hydroformylierungsergebnisse beider Liganden unter vergleichbaren Bedingungen dargestellt. Dabei geht deutlich hervor, dass sich der symmetrische Biphephosligand (in der Literaturstelle Ligand 5a) durch eine deutlich höhere n/i-Selektivität und eine höhere Aktivität auszeichnet als sein unsymmetrisches Isomer (in der Literaturstelle Ligand 7). In der Hydroformylierungsreaktion von Propen weist der symmetrische Ligand eine n/i-Selektivität von 53 und eine Reaktionsrate von 402 auf, wohingegen der unsymmetrische Ligand lediglich eine n/i-Selektivität von 1.2 und eine Reaktionsrate von 280 auf. Dies konnte durch die eigenen Ergebnisse in Tabelle 3 noch einmal bestätigt werden.

Weiterhin wurden der erfindungsgemäße Ligand (1) und sein symmetrisches Isomer (10) unter vergleichbaren Bedingungen getestet. Die nachfolgenden Versuche wurden nach der Vorschrift in Beispiel 2 durchgeführt. Lediglich die Liganden wurden ausgetauscht. In Tabelle 4 sind die Hydroformylierungsergebnisse von cis-2-Buten mit dem erfindungsgemäßen Liganden (1) und seinem symmetrischen Isomeren, Ligand (10), dargestellt.

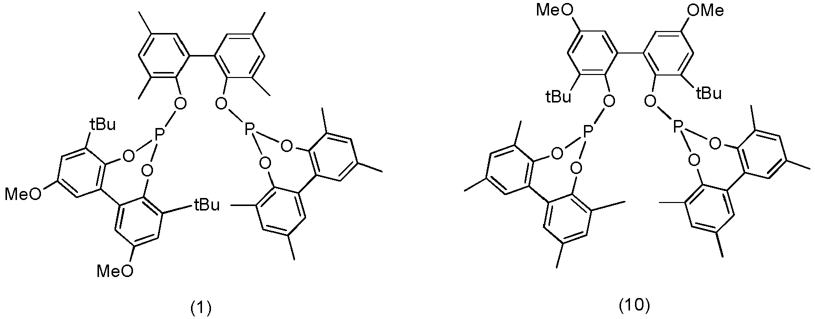

**Tabelle 4:**

| Eintrag | Ligand | mol% Pentanal | mol% 2-Methylbutanal | Regioselektivität n-Pentanal in % |
|---|---|---|---|---|
| 1 | (1) | 80,0 | 5,2 | 94,0 |
| 2 | (10) | 59,9 | 6,35 | 90,4 |

Der erfindungsgemäße unsymmetrische Ligand (1) (Eintrag 1) weist eine sehr gute n-Pentanal-Regioselektivität von 94% und gute Aldehydausbeuten auf. Sein symmetrisches Isomer (Eintrag 2) hingegen weist geringere Pentanalselektivitäten von lediglich 90% und deutlich geringere Aktivitäten, d.h. Ausbeuten aus.
Dieses Ergebnis ist überraschend, da unsymmetrische substituierte Bisphosphite gegenüber symmetrisch substituierten deutlich an Aktivität und Selektivität einbüßen, wie im Stand der Technik beschrieben und durch die obigen Vergleichsversuche mit dem Liganden Biphephos und seinem unsymmetrischen Isomer (9) bestätigt. Somit zeichnet sich der erfindungsgemäße unsymmetrische Ligand (1) völlig entgegen dem Stand der Technik durch sehr gute Selektivitäten und Aktivitäten aus. Weiterhin handelt es sich bei dem erfindungsgemäßen Liganden (1) um einen sehr langzeitstabilen Liganden wie in dem nachfolgenden Langzeitversuch in einer kontinuierlich betriebenen Anlage gezeigt wird.

### Vergleichsversuch - Langzeitversuch

In einer ersten Versuchsreihe wurde die erfindungsgemäße Verbindung (1) getestet. In der zweiten Versuchsreihe wurde unter den gleichen Versuchsbedingungen die Vergleichsverbindung Biphephos verwendet:

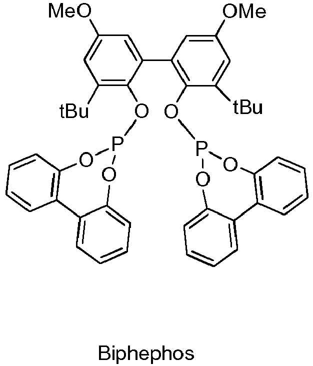

Eine Hydroformylierung von Buten/Butan-Mischungen wurde in einer kontinuierlich betriebenen Versuchsanlage durchgeführt.
Diese Versuchsanlage bestand im Wesentlichen aus einem 20 Liter fassenden Druckreaktor mit einem nachgeschalteten Kondensator und Phasentrennbehälter (Gas/Flüssigkeit) für die aus dem Reaktor stammende Gasphase sowie einem Kreisgasverdichter, der die Gasphase aus dem Phasentrennbehälter wieder unten in die Reaktionszone zurück führt. Ein Teil dieses Kreisgases wird nach der Phasentrennung als Abgas aus dem Reaktionssystem gefahren.
Um eine optimale Gasverteilung im Reaktorsystem zu realisieren, wurde hier ein Gasverteilerring mit Bohrungen verbaut.
Über installierte Heiz- und Kühlvorrichtungen konnte der Reaktor temperiert werden.

Vor der Hydroformylierung wurde das Reaktorssystem mit Stickstoff frei von Sauerstoff gespült. Anschließend wurde der Reaktor mit 12 Liter Katalysatorlösung gefüllt.
Diese Katalysatorlösung setzte sich aus 12 kg Isononylbenzoat, 4.5 g Rh(acac)(CO)₂, 63 g Bisphosphit-Ligand (1), 200g Amin IIb zusammen und wurde vorher in einem Behälter gemischt. Das Isononylbenzoat wurde zuvor mit Stickstoff gestrippt, um Sauerstoff und Wasser aus dem Lösemittel zu entfernen.
Anschließend wurde das Reaktorsystem mit Synthesegas frei von Stickstoff gespült. Nachdem der Stickstoffgehalt < 10 Vol% gefallen war, wurde das Reaktorsystem mit Synthesegas auf 1,0 MPa aufgedrückt und anschließend auf 120 °C aufgeheizt.
Nach Erreichen der Betriebstemperatur wurde das Reaktorsystem mit Synthesegas auf 1,7 MPa Reaktionsdruck gebracht.
Danach wurde die Zugabe der Ausgangsstoffe gestartet. Das Rohbutan wurde über einen Verdampfer gefahren, um das Rohbutan gasförmig in das Kreisgas zu fahren.

Folgende Durchsätze wurden eingestellt:
0,3 kg/h Rohbutan (eine Mischung aus 35 % 2-Butenen und n-Butan und 1-Buten Konzentrationen von ca. 1 %) 75 NI/h Synthesegas (50 Vol% H2 und 50 Vol% CO). Zur täglichen Dosierung der Verbindung (1) und Amins IIb wurde eine 1,4%ige Lösung des Bisphosphit-Liganden I in n-Pentanal angesetzt, welches zuvor durch Strippen mit Stickstoff von restlichen C4-Kohlenwasserstoffen (< 3 %) befreit wurde. Das Amin IIb wurde in einem dreifachen molaren Überschuss zur Verbindung (1) eingesetzt. Zur besseren Stabilisierung dieser Lösung wurde das Amin IIb vor dem Bisphosphit-Liganden (1) zur Lösung gegeben.

Die Reaktionsprodukte wurden kontinuierlich über den Kreisgasstrom aus dem Reaktor entfernt und im Kondensator bei 50 °C partiell auskondensiert. Die auskondensierte Phase wurde kontinuierlich aus dem Phasentrennbehälter gefahren. Zur Ausbeutebestimmung wurden aus dem Kreisgas vor und nach Reaktor Proben gezogen und mittels Gaschromatograph analysiert.
Durch eine tägliche Dosierung der oben beschriebenen Ligandenlösung, konnte der Umsatz und die Regioselektivität konstant gehalten werden.
Zur Bestimmung des Reaktorinhaltes wurden Proben aus dem Reaktor entnommen und mittels Flüssigchromatographie (HLPC) untersucht.

Unter den gewählten Reaktionsbedingungen stellte sich eine Aldehydausbeute zwischen 80% und 90% ein. Dieser Zustand konnte bis zum Versuchsende konstant gehalten werden. Die prozentuale Verteilung zwischen n-Pentanal und 2-Methylbutanal, bzw. die Regio-Selektivität, betrug 92 % zu 8 %.
In der stationären Phase des Versuches konnte kein Rhodiumabbau verzeichnet werden.

Die Ergebnisse sind in Figur 3 dargestellt.
Figur 3 zeigt einen 1500 h Langzeitversuch in der Hydroformylierung von Rohbutan mit dem erfindungsgemäßen unsymmetrischen Liganden (1). Während der gesamten Versuchsdauer konnte eine konstant hohe Aktivität, d.h. Aldehydausbeute von durchschnittlich 80% gewährleistet werden bei weiterhin sehr guter Regioselektivität.
In der zweiten Versuchsreihe wurde anstelle der erfindungsgemäßen Verbindung (1) 55 g der Vergleichsverbindung Biphephos eingesetzt. Die Ergebnisse sind in Figur 4 dargestellt.
Unter den gewählten Reaktionsbedingungen fiel die Aldehydausbeute von anfänglich 70 % bis 80% nach 150 h auf 40% bis 50% ab. Die prozentuale Verteilung zwischen n-Pentanal und 2-Methylbutanal, bzw. die Regio-Selektivität, betrug 95 % zu 5 %.
In der stationären Phase des Versuches konnte kein Rhodiumabbau verzeichnet werden. Figur 4 zeigt einen 250 h Langzeitversuch in der Hydroformylierung von Rohbutan mit dem symmetrischen Vergleichsliganden Biphephos. Hierbei kann im Vergleich zu dem erfindungsgemäßen Liganden keine langandauernde Aktivität gewährleistet werden. Unter den gewählten Reaktionsbedingungen fiel die Aldehydausbeute von anfänglich 70 % bis 80% nach 150 h auf 40% bis 50% ab. Die Regio-Selektivität war weiterhin sehr gut. D.h dieser Ligand zeichnet sich durch eine deutlich geringere Langzeitstabilität.

Folglich zeichnet sich der erfindungsgemäße unsymmetrische Ligand (1) durch eine deutlich verbesserte Stabilität aus als der symmetrische Vergleichsligand Biphephos. Der erfindungsgemäße Ligand (1) in der katalytisch aktiven Zusammensetzung zeichnet sich durch eine deutlich bessere Langzeitstabilität als die bisher im Stand der Technik beschriebenen Liganden aus und erfüllt somit die gestellte Aufgabe. Eine optimale Langzeitstabilität der katalytisch aktiven Zusammensetzung ist insbesondere in der großtechnischen Anwendung von Bedeutung, da der Ligand in der Hydroformylierungsreaktion großtechnisch zwar nachdosiert werden kann, jede Nachdosierung jedoch die Wirtschaftlichkeit eines großtechnischen Prozesses negativ beeinflusst und ihn ggf. unrentabel macht. Somit ist es essentiell einen möglichst langzeitstabilen Liganden einzusetzen, der sich neben der Langzeitstabilität auch durch eine gute Aktivität und eine gute n/i-Selektivität auszeichnet. Diese Aufgabe wird durch den erfindungsgemäßen Liganden (1) erfüllt.

## Patentansprüche

1. Verbindung der Formel (1):

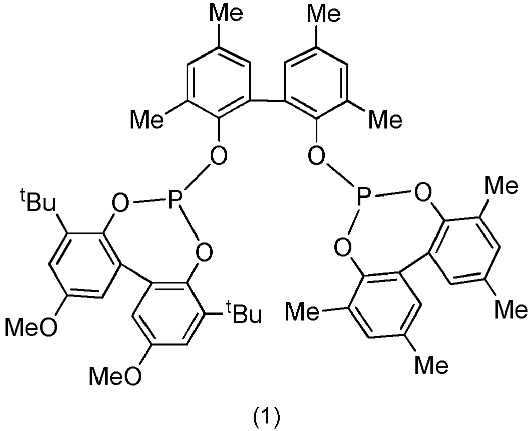

2. Verfahren zur Herstellung einer Verbindung nach Anspruch 1 umfassend die Verfahrensschritte:
i) oxidative Kopplung von 2,4-Dimethylphenol zu 3, 3', 5, 5'-Tetramethyl-2,2'-dihydroxybiphenyl;
ii) oxidative Kopplung von 3-tert.-Butyl-4-Hydroxyanisol zu 5, 5'-Dimethoxy-3, 3'-di-tert.-Butyl-2, 2'-dihydroxybiphenyl;
iii) Umsetzung von 3, 3', 5, 5'-Tetramethyl-2,2'-dihydroxybiphenyl aus i) mit PCl₃ zum Phosphorochloriditderivat unter Inertgasatmosphäre;
iv) Umsetzung von zumindest 2 Äquivalenten des Phophorochloriditderivats aus iii) mit 1 Äquivalent des 5, 5'-Dimethoxy-3, 3'-di-tert.-Butyl-2, 2'-dihydroxybiphenyl aus ii) unter Inertgasatmosphäre.

3. Verfahren nach Anspruch 2,
wobei im Verfahrensschritt iv) ein Lösungsmittelgemisch verwendet wird.

4. Verfahren nach Anspruch 3,
wobei das Lösungsmittelgemisch, welches im Verfahrensschritt iv) verwendet wird, ausgewählt ist aus organischen Stickstoffverbindungen, organischen Estern, Aromaten.

5. Verfahren nach Anspruch 4,
**dadurch gekennzeichnet, dass** die organische Stickstoffverbindung eine Verbindung ist ausgewählt unter Nitrilen, Aminen, Amiden.

6. Verfahren nach einem der Ansprüche 2 bis 5,
zusätzlich umfassend den Verfahrensschritt v), indem die Verbindung (1) als Feststoff abgetrennt und in einem aprotischen Lösungsmittelgemisch suspendiert und/oder umkristallisiert wird.

7. Verbindung gemäß der Formel (2): 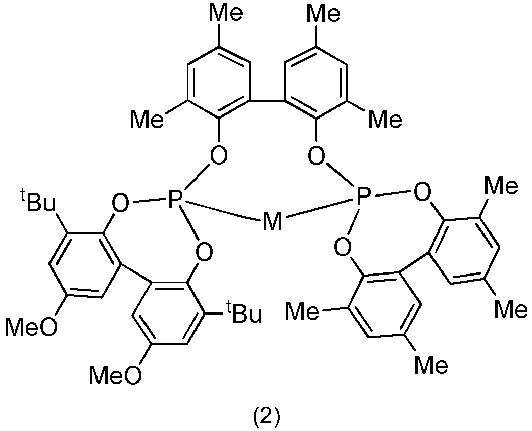 wobei M ausgewählt ist aus Fe, Ru, Os, Co, Rh, Ir, Ni, Pd, Pt und M zusätzliche Bindungen eingehen kann.

8. Verbindung gemäß der Formel (3): 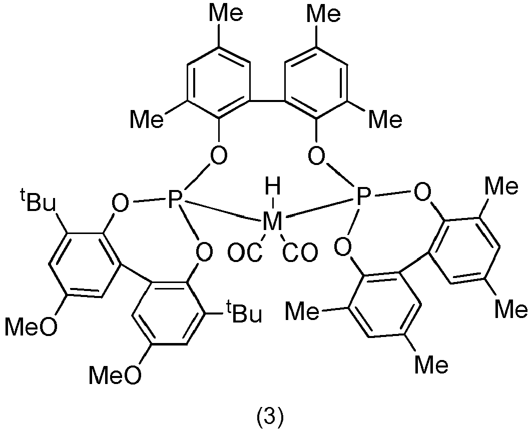 wobei M ausgewählt ist aus: Fe, Ru, Os, Co, Rh, Ir, Ni, Pd, Pt.

9. Verbindung nach Anspruch 8, wobei M Rh ist.

10. Gemisch umfassend eine Verbindung gemäß der Formel (2) und/oder (3),
wobei das Gemisch zusätzlich eine Verbindung gemäß der Formel (1) umfasst, welche nicht an M gebunden ist.

11. Zusammensetzung umfassend ein Gemisch nach Anspruch 10,
welche zusätzlich zu dem Gemisch eine weitere Komponente aufweist ausgewählt aus: Basen, organische Amine, Pufferlösungen, Epoxide, Ionenaustauscher.

12. Zusammensetzung nach Anspruch 11,
wobei das organische Amin zumindest eine 2,2,6,6-Tetramethylpiperidineinheit aufweist.

13. Zusammensetzung nach Anspruch 11,
wobei die weitere Komponente ein Sebacinsäuredi-4-(2,2,6,6-tetramethylpiperidinyl)-ester ist.

14. Verfahren zur Hydroformylierung einer ungesättigten Verbindung oder eines Gemisches von ungesättigten Verbindungen umfassend die Verfahrensschritte:
a) Vorlegen einer Verbindung nach Anspruch 1 oder einer Zusammensetzung nach den Ansprüchen 11 bis 13,
b) Einleiten eines Gasgemisches umfassend Kohlenmonoxid und Wasserstoff
c) Zugabe mindestens einer ungesättigten Verbindung oder eines Gemisches von ungesättigten Verbindungen.

15. Verfahren nach Anspruch 14,
wobei die ungesättigte Verbindung oder deren Gemisch ausgewählt sind aus:
- Kohlenwasserstoffgemischen aus Dampfspaltanlagen;
- Kohlenwasserstoffgemischen aus katalytisch betriebenen Spaltanlagen;
- Kohlenwasserstoffgemischen aus Oligomerisierungsprozessen;
- Kohlenwasserstoffgemischen umfassend mehrfach ungesättigte Verbindungen;
- ungesättigten Carbonsäurederivaten.

16. Verfahren nach Anspruch 14 oder 15,
**dadurch gekennzeichnet, dass** das Gemisch ungesättigte Verbindungen mit 2 bis 30 Kohlenstoffatomen aufweist.

17. Verfahren nach einem der Ansprüche 14 bis 16,
**dadurch gekennzeichnet, dass** das Gemisch ungesättigte Verbindungen mit 2 bis 8 Kohlenstoffatomen aufweist.

18. Verfahren nach einem der Ansprüche 14 bis 17,
**dadurch gekennzeichnet, dass** die ungesättigten Carbonsäurederivate ausgewählt sind unter Fettsäureestern.

## Claims

1. Compound of the formula (1):

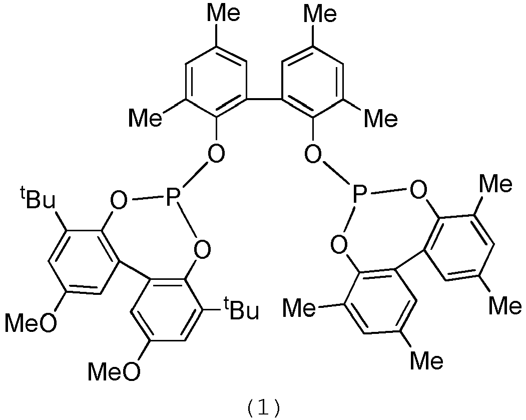

2. Process for preparing a compound according to Claim 1, comprising the process steps of:
i) oxidatively coupling 2,4-dimethylphenol to give 3,3',5,5'-tetramethyl-2,2'-dihydroxybiphenyl;
ii) oxidatively coupling 3-tert-butyl-4-hydroxyanisole to give 5,5'-dimethoxy-3,3'-di-tert-butyl-2,2'-dihydroxybiphenyl;
iii) reacting 3,3',5,5'-tetramethyl-2,2'-dihydroxybiphenyl from i) with PCl₃ to give the phosphorochloridite derivative under inert gas atmosphere;
iv) reacting at least 2 equivalents of the phosphorochloridite derivative from iii) with 1 equivalent of the 5,5'-dimethoxy-3,3'-di-tert-butyl-2,2'-dihydroxybiphenyl from ii) under inert gas atmosphere.

3. Process according to Claim 2,
wherein a solvent mixture is used in process step iv).

4. Process according to Claim 3, wherein the solvent mixture which is used in process step iv) is selected from organic nitrogen compounds, organic esters, aromatics.

5. Process according to Claim 4, **characterized in that** the organic nitrogen compound is a compound selected from nitriles, amines, amides.

6. Process according to any of Claims 2 to 5, additionally comprising process step v), in which the compound (1) is removed in solid form and suspended and/or recrystallized in an aprotic solvent mixture.

7. Compound of the formula (2): 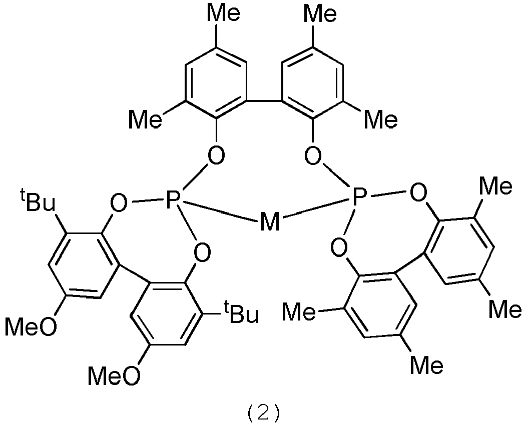 where M is selected from Fe, Ru, Os, Co, Rh, Ir, Ni, Pd, Pt, and M may enter into additional bonds.

8. Compound of the formula (3): 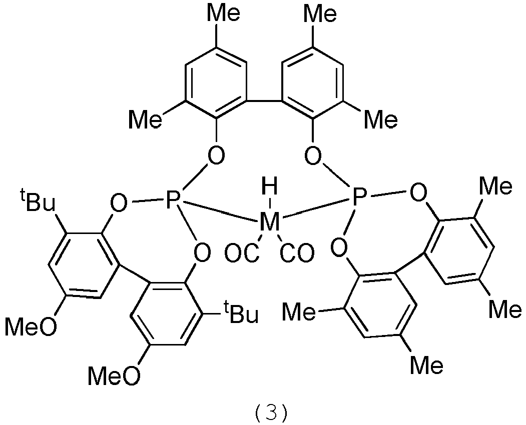 where M is selected from: Fe, Ru, Os, Co, Rh, Ir, Ni, Pd, Pt.

9. Compound according to Claim 8, where M is Rh.

10. Mixture comprising a compound of the formula (2) and/or (3),
where the mixture additionally comprises a compound of the formula (1) not bonded to M.

11. Composition comprising a mixture according to Claim 10,
including, in addition to the mixture, a further component selected from: bases, organic amines, buffer solutions, epoxides, ion exchangers.

12. Composition according to Claim 11,
wherein the organic amine has at least one 2,2,6,6-tetramethylpiperidine unit.

13. Composition according to Claim 11, wherein the further component is a di-4-(2,2,6,6-tetramethylpiperidinyl) sebacate.

14. Process for hydroformylating an unsaturated compound or a mixture of unsaturated compounds, comprising the process steps of:
a) initially charging a compound according to Claim 1 or a composition according to Claims 11 to 13,
b) introducing a gas mixture comprising carbon monoxide and hydrogen
c) adding at least one unsaturated compound or a mixture of unsaturated compounds.

15. Process according to Claim 14,
wherein the unsaturated compound or mixture thereof are selected from:
- hydrocarbon mixtures from steamcracking plants;
- hydrocarbon mixtures from catalytically operated cracking plants;
- hydrocarbon mixtures from oligomerization operations;
- hydrocarbon mixtures comprising polyunsaturated compounds;
- unsaturated carboxylic acid derivatives.

16. Process according to Claim 14 or 15, **characterized in that** the mixture includes unsaturated compounds having 2 to 30 carbon atoms.

17. Process according to any of Claims 14 to 16, **characterized in that** the mixture includes unsaturated compounds having 2 to 8 carbon atoms.

18. Process according to any of Claims 14 to 17, **characterized in that** the unsaturated carboxylic acid derivatives are selected from fatty acid esters.

## Revendications

1. Composé de formule (1) :

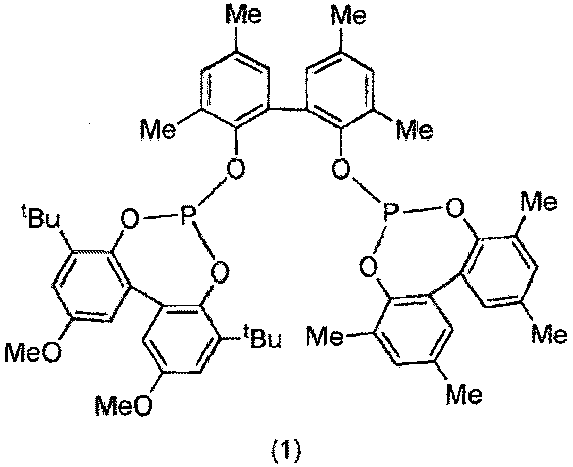

2. Procédé de fabrication d'un composé selon la revendication 1, comprenant les étapes de procédé suivantes :
i) le couplage oxydatif de 2,4-diméthylphénol en 3,3',5,5'-tétraméthyl-2,2'-dihydroxybiphényle ;
ii) le couplage oxydatif de 3-tert.-butyl-4-hydroxyanisol en 5,5'-diméthoxy-3,3'-di-tert.-butyl-2,2'-dihydroxybiphényle ;
iii) la mise en réaction de 3,3',5,5'-tétraméthyl-2,2'-dihydroxybiphényle de i) avec PCl₃ pour former le dérivé phosphorochloridite sous une atmosphère de gaz inerte ;
iv) la mise en réaction d'au moins 2 équivalents du dérivé phosphorochloridite de iii) avec 1 équivalent du 5,5'-diméthoxy-3,3'-di-tert.-butyl-2,2'-dihydroxybiphényle de ii) sous une atmosphère de gaz inerte.

3. Procédé selon la revendication 2, dans lequel un mélange de solvants est utilisé à l'étape de procédé iv).

4. Procédé selon la revendication 3, dans lequel le mélange de solvants qui est utilisé à l'étape de procédé iv) est choisi parmi les composés azotés organiques, les esters organiques, les composés aromatiques.

5. Procédé selon la revendication 4, **caractérisé en ce que** le composé azoté organique est un composé choisi parmi les nitriles, les amines, les amides.

6. Procédé selon l'une quelconque des revendications 2 à 5, comprenant en outre l'étape de procédé v), lors de laquelle le composé (1) est séparé sous forme solide et suspendu et/ou recristallisé dans un mélange de solvants aprotique.

7. Composé selon la formule (2) : 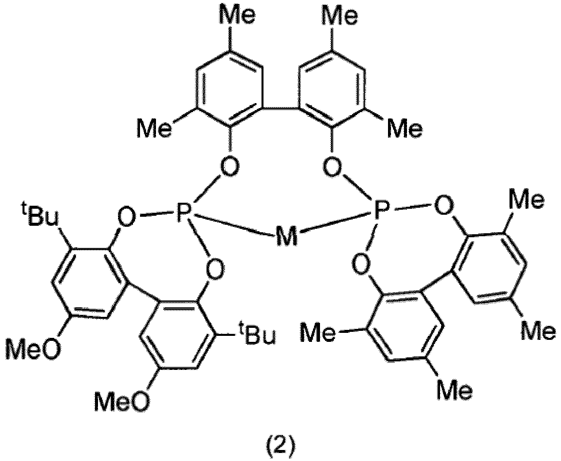 dans laquelle M est choisi parmi Fe, Ru, Os, Co, Rh, Ir, Ni, Pd, Pt et M peut former des liaisons supplémentaires.

8. Composé selon la formule (3) : 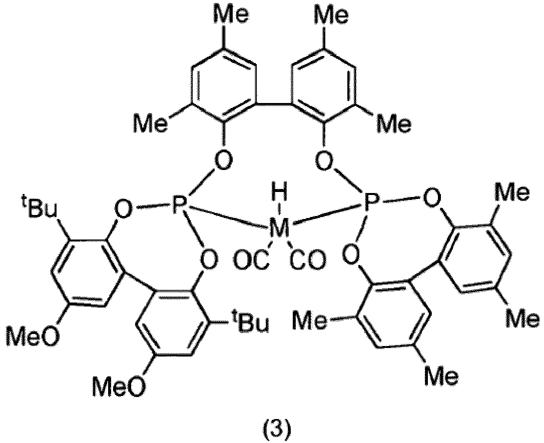 dans laquelle M est choisi parmi : Fe, Ru, Os, Co, Rh, Ir, Ni, Pd, Pt.

9. Composé selon la revendication 8, dans lequel M est Rh.

10. Mélange comprenant un composé selon la formule (2) et/ou (3), le mélange comprenant en outre un composé selon la formule (1), qui n'est pas relié à M.

11. Composition comprenant un mélange selon la revendication 10, qui comprend en plus du mélange un composant supplémentaire choisi parmi : les bases, les amines organiques, les solutions tampons, les époxydes, les échangeurs d'ions.

12. Composition selon la revendication 11, dans laquelle l'amine organique comprend au moins une unité 2,2,6,6-tétraméthylpipéridine.

13. Composition selon la revendication 11, dans laquelle le composant supplémentaire est un ester di-4-(2,2,6,6-tétraméthylpipéridinylique) de l'acide sébacique.

14. Procédé d'hydroformylation d'un composé insaturé ou d'un mélange de composés insaturés, comprenant les étapes de procédé suivantes :
a) le chargement d'un composé selon la revendication 1 ou d'une composition selon les revendications 11 à 13,
b) l'introduction d'un mélange gazeux comprenant du monoxyde de carbone et de l'hydrogène,
c) l'ajout d'au moins un composé insaturé ou d'un mélange de composés insaturés.

15. Procédé selon la revendication 14, dans lequel le composé insaturé ou le mélange de composés insaturés est choisi parmi :
- les mélanges d'hydrocarbures issus d'unités de clivage à la vapeur ;
- les mélanges d'hydrocarbures issus d'unités de clivage catalytiques ;
- les mélanges d'hydrocarbures issus de procédés d'oligomérisation ;
- les mélanges d'hydrocarbures comprenant des composés polyinsaturés ;
- les dérivés d'acides carboxyliques insaturés.

16. Procédé selon la revendication 14 ou 15, **caractérisé en ce que** le mélange comprend des composés insaturés de 2 à 30 atomes de carbone.

17. Procédé selon l'une quelconque des revendications 14 à 16, **caractérisé en ce que** le mélange comprend des composés insaturés de 2 à 8 atomes de carbone.

18. Procédé selon l'une quelconque des revendications 14 à 17, **caractérisé en ce que** les dérivés d'acides carboxyliques insaturés sont choisis parmi les esters d'acides gras.
